# EUROPEAN PATENT APPLICATION

(11) **EP 3 444 333 A1**
(43) Date of publication of application: **20.02.2019**
(21) Application number: 18174732.0
(22) Date of filing: 22.10.2010
(51) Int. Cl.: C12N 5/0783

(54) **CELL-BASED ANTI-CANCER COMPOSITIONS AND METHODS OF MAKING AND USING THE SAME**

(30) Priority: 22.10.2009 US 25411909 P
(62) Divisional of application: 10833742.9
(71) Applicant: Thomas Jefferson University, Philadelphia, PA 19107 (US)
(72) Inventor: WALDMAN, Scott A., Philadelphia, PA 19107 (US); SNOOK, Adam E., Aston, PA 19014 (US)
(74) Representative: Leonard, Thomas Charles

(57) **Abstract**

Isolated pluralities of T cells which recognize at least one epitope of a mucosally restricted antigen and pharmaceutical compositions comprising the same are disclosed. Methods of making a plurality of T cells that recognize at least one epitope of a mucosally restricted antigen are also disclosed. Methods of treating an individual who has been diagnosed with cancer of a mucosal tissue or preventing such cancer in an individual at elevated risk are disclosed as are nucleic acid molecules that comprise a nucleotide sequence that encode proteins that recognize at least one epitope of a mucosally restricted antigen and T cells comprising such nucleic acid molecules.

## Description

### FIELD OF THE INVENTION

The invention relates to compositions that comprise T cells that target mucosal tissue-derived antigens, to methods of making such compositions, and to methods of using them to protect individuals against metastatic cancer whose origin is a mucosal tissue and for treating individuals who are suffering from metastatic cancer whose origin is a mucosal tissue.

### BACKGROUND OF THE INVENTION

Despite improvements and successes in therapy, cancer continues to claim the lives of numerous people worldwide. For example, colorectal cancer is the third most common cause of death from malignant disease in Western countries. Worldwide, it has been estimated there are at least half a million new cases of colorectal cancer each year.

Improvements in screening provide the opportunity to identify many individuals who have early stage cancer as well as many who do not have cancer but who are genetically predisposed to developing cancer and thus at an elevated risk of developing cancer. Moreover, because of improvements in treatment, there are numerous people who have either had cancer removed or in remission. Such people are at a risk of relapse or recurrence and so are also at an elevated risk of developing cancer.

There is a need for improved methods of treating individuals suffering from cancer of mucosal tissue. There is a need for compositions useful to treat individuals suffering from cancer of mucosal tissue. There is a need for improved methods of preventing a recurrence of cancer of mucosal tissue in individuals who have been treated for cancer of mucosal tissue. There is a need for compositions useful to prevent a recurrence of cancer of mucosal tissue in individuals who have been treated for cancer of mucosal tissue. There is a need for improved methods of preventing cancer of mucosal tissue in individuals, particularly those who have been identified as having a genetic predisposition for cancer of mucosal tissue. There is a need for compositions useful for preventing cancer of mucosal tissue in individuals. There is a need for improved methods of identifying compositions useful to treat and prevent cancer of mucosal tissue in individuals.

### SUMMARY OF THE INVENTION

Isolated pluralities of T cells which recognize at least one epitope of a mucosally restricted antigen are provided.

Pharmaceutical compositions comprising an isolated plurality of T cells which recognize at least one epitope of a mucosally restricted antigen and a pharmaceutically acceptable carrier or diluent are also provided.

Methods of making a plurality of T cells that recognize at least one epitope of a mucosally restricted antigen are provide. Some methods comprise the steps of isolating a sample from a cell donor that comprises at least one T cell that recognize at least one epitope of a mucosally restricted antigen; identifying a T cell that recognize at least one epitope of a mucosally restricted antigen; and culturing said T cell under conditions to promote its replication for a period sufficient to produce a plurality of T cells that recognize at least one epitope of a mucosally restricted antigen. Other methods comprise the steps of isolating a sample from a cell donor that comprises at least one T cell; transforming the T cell with a nucleic acid sequence that encodes either a T cell receptor that recognizes at least one epitope of a mucosally restricted antigen, or a cancer mucosal antigen-binding membrane-bound fusion protein that comprises at least a functional fragment of an antibody that binds to at least one epitope of a mucosally restricted antigen, wherein upon expression the fusion protein is a membrane bound protein; and culturing said transformed T cell under conditions to promote its replication for a period sufficient to produce plurality of T cells that recognize at least one epitope of a mucosally restricted antigen.

Methods of treating an individual who has been diagnosed with cancer of a mucosal tissue are provided which comprise the step of administering to the individual an effective amount of a plurality of T cells that recognize at least one epitope of a mucosally restricted antigen.

Methods of preventing cancer of a mucosal tissue in an individual identified as being at an elevated risk of developing cancer of a mucosal tissue comprising the step of administering to the individual an effective amount of a plurality of T cells which recognize at least one epitope of a mucosally restricted antigen are also provided.

In addition nucleic acid molecules are provided which comprise a nucleotide sequence that encodes a T cell receptor protein that recognizes at least one epitope of a mucosally restricted antigen or a cancer mucosal antigen-binding membrane-bound fusion protein that comprises coding sequence for at least a functional fragment of an antibody which binds to at least one epitope of a mucosally restricted antigen and a portion which renders the fusion protein a membrane bound protein when the nucleotide sequence is expressed in a cell.

T cells comprising such nucleic acid molecules are also provided.

### DETAILED DESCRIPTION OF THE INVENTION

### Definitions

As used herein, "mucosal tissue" refers to tissue of the mucosa which is moist tissue that lines some organs and body cavities throughout the body, including the nose, mouth, lungs, and digestive tract. Mucosal tissue may be found in several different parts of the body, including but not limited to: the mouth, such as buccal, sublingual and oral mucosal tissue; the nose, such as olfactory mucosal tissue; the lungs; the digestive tract, such as the esophagus, the stomach, the duodenum, the small and large intestines, the colon, the rectum and the anus; and the uro-genital organs such as the bladder, urethra, parts of the vagina, parts of the penis and the uterus. Mucosal tissue is also found as part of the breast, kidney and eyes.

As used herein, "an individual is suspected of being susceptible to cancer of mucosal tissue" is meant to refer to an individual who is at an above-average risk of developing cancer of mucosal tissue. Examples of individuals at a particular risk of developing cancer of mucosal tissue are those whose family medical history indicates above average incidence of cancer of mucosal tissue among family members and/or those who have genetic markers whose presence is correlatively for elevated incidence of mucosal cancer and/or those who have already developed cancer of mucosal tissue and have been treated who therefore face a risk of disease progression, relapse or recurrence. Factors which may contribute to an above-average risk of developing cancer of mucosal tissue which would thereby lead to the classification of an individual as being suspected of being susceptible to cancer of mucosal tissue may be based upon an individual's specific genetic, medical and/or behavioral background and characteristics.

As used herein, "a mucosally-restricted antigen" is meant to refer to an antigen which is expressed in normal mucosal cells but not normal non-mucosal cells. Examples of mucosally-restricted antigen include guanylyl cyclase C, CDX-1, CDX-2, sucrase isomaltase, mammoglobin, small breast epithelial mucin, intestine specific homeobox, RELM beta (FIZZ2), Villin, A33, Lactase (lactase-phlorizin hydrolase), H(+)/peptide cotransporter 1 (PEPT1, SLC15A1), Intectin, Carbonic anhydrase, Mammaglobin, B726P, small breast epithelial mucin (SBEM), LUNX, and TSC403.

As used herein the term "isolated plurality of T cells that recognizes at least one epitope of a mucosally restricted antigen" refers to a population of at least 1 x 106 T cells maintained outside a living organism which are each reactive to at least one epitope of a mucosally restricted antigen. In some embodiments, the isolated plurality of T cells comprises a population of at least 1 x 107 such T cells maintained outside a living organism. In some embodiments, the isolated plurality of T cells comprises a population of at least 1 x 108 such T cells maintained outside a living organism. In some embodiments, the isolated plurality of T cells comprises a population of at least 1 x 109 such T cells maintained outside a living organism. In some embodiments, the isolated plurality of T cells comprises a population of at least 1 x 1010 such T cells maintained outside a living organism. In some embodiments, the isolated plurality of T cells comprises a population of at least 1 x 1011 such T cells maintained outside a living organism. A population may be maintained in multiple containers or a single container.

As used herein the term "at least one epitope of a mucosally restricted antigen" is meant to refer to a molecule that is immunologically cross reactive with a mucosally restricted antigen including the full length mucosally restricted antigen and fragments thereof.

As used herein the term "a CMA-binding membrane bound fusion protein" refers to a protein comprises at least a functional fragment of an antibody that recognizes at least one epitope of a mucosally restricted antigen and a portion which causes the protein, when expressed in a T cell, to be a membrane bound protein.

As used herein, "a CD4+ helper epitope" is peptide sequence that forms a complex with a Major Histocompatibility Complex (MHC) Class 2 human leukocyte antigen (HLA) and is recognized by T cell receptors on CD4+ T cells. A peptide, e.g. CD4+ helper epitope, forms a complex with an MHC and this complex may be recognized by a particular T cell receptor. The interaction between the MHC/peptide complex and the T cell receptor results in signals between the cell expressing the MHC and the T cell expressing the T cell receptor. In the case of the MHC class II, the complex formed by the peptide and MHC class II complex interacts with T cell receptors of CD4+ helper T cells. Thus, a peptide which can form a complex with an MHC class II molecule that can be recognized as a complex by a T cell receptor of a CD4+ helper T cell is a CD4+ helper epitope.

As used herein, "a secretion signal" and "a secretion peptide" and "a signal peptide" are used interchangeably and meant to refer to an amino acid sequence of a protein which when present results in the transportation and secretion of the protein to the exterior of the cell. Secretion signals are typically cleavable hydrophobic segments of a precursor protein at or near the N terminus of the precursor protein. In the secretion process, such secretion signals are enzymatically removed to result in the secretion of a mature form of the protein, i.e. a form of the protein lacking the secretion signal. In some embodiments, the secretion signal is derived from the mucosally restricted antigen. In some embodiments, the secretion signal is derived from another source. Examples of secretion signals include those which are present on the mucosally restricted antigen or those derived from other sources. In the case of the former, the coding sequence of the mucosally restricted antigen including the signal sequence is used intact. In the case of the latter, a nucleotide sequence encoding the signal sequence is linked the coding sequence of the mucosally restricted antigen. In such cases, the signal sequence may be any such sequence which is functional in the cells of the individual to whom the genetic construct is administered.

### Overview

U.S. Provisional Serial Number 61/099,398, which is incorporated herein by reference, refers to vaccines that target a class of vaccine targets for tumors arising from mucosa (aerodigestive, urogenital, breast, other), termed cancer mucosal antigens (CMAs). U.S. Published Patent Application No. 20040141990 published July 22, 2004, which is incorporated herein by reference, refers to vaccines for metastatic colorectal cancer. U.S. Published Patent Application Nos. 20010039017, 20010036635, 20010029020, and 20010029019, which are each incorporated herein by reference, each refer to vaccines that targeting cancer cells of alimentary canal origin.

CMAs, also referred to herein as mucosally restricted antigens, are normally expressed only in the mucosal compartment and their expression persists after mucosal cells undergo neoplastic transformation and become cancer cells. Moreover, these antigens continue to be expressed after these tumor cells metastasize. There are several advantages in using these antigens as immunotherapeutic targets. There may be only partial tolerance in the systemic compartment, which is normally naive to these antigens, permitting systemic treatment which provides anti-metastatic tumor efficacy. Further, there is an absence of cross compartmental immune responses which may provide an avoidance mucosal inflammation and autoimmunity.

A protein comprising at least one epitope of a mucosally restricted antigen, such as a full length mucosally restricted antigen or fragment thereof is immunogenically crossreactive with the mucosally restricted antigen of a cancer of mucosal tissue.

Immunization of some cancer patients has been observed to be suboptimal reflecting changes in immunity associated with age, disease and genetic polymorphisms among the population and by limitations on generating immune responses to a self protein. Instead of immunizing patients to produce a T cell immune response, T cells large numbers of specific T cells may be administered to patients.

T cells are provided which recognize at least one epitope of a mucosally restricted antigen such that these T cells will bind to mucosal cancer cells which express the mucosally restricted antigen, and thereby immunologically react with and against the cancer cells. While not wishing to be limited to any particular method of making pluralities of T cells which recognize at least one epitope of a mucosally restricted antigen, three methods are provided herein. A first way to obtain a plurality of T cells which recognize at least one epitope of a mucosally restricted antigen is to isolate a T cell which recognize at least one epitope of a mucosally restricted antigen and, using culturing techniques, exponentially expand the number of T cells to produce a plurality of such cells. A second way to obtain a plurality of T cells which recognize at least one epitope of a mucosally restricted antigen is to isolate a T cell from an individual, transform it with a nucleic acid molecule that encodes a T cell receptor which recognizes at least one epitope of a mucosally restricted antigen and, using culturing techniques, exponentially expand the number of transformed T cells to produce a plurality of such cells. A third way to obtain a plurality of T cells which recognize at least one epitope of a mucosally restricted antigen is to isolate a T cell from an individual, transform it with a nucleic acid molecule that encodes a fusion protein which includes a functional fragment of an antibody that binds to at least one epitope of a mucosally restricted antigen and a portion that renders the protein, when expressed in a cell such as a T cell, a membrane bound protein.

These T cells are used as therapeutics and prophylactics against cancer of the mucosal tissue which comprises cells that express the mucosally restricted antigen.

### Anti-CMA T cells as starting material

The clonal expansion of a T cell that recognizes at least one epitope of a mucosally restricted antigen comprises isolating such a T cell from a cell donor and, using culturing techniques, exponentially expand the number of cells by maintaining them under conditions which promote cell division.

The cell donor may be the individual to whom the expanded population of cells will be administered, i.e. an autologous cell donor. Alternatively, the T cell may be obtained from a cell donor that is a different individual from the individual to whom the T cells will be administered, i.e. an allogenic T cell. If an allogenic T cell is used, it is preferred that the donor be type matched, that is identified as expressing the same or nearly the same set fo leukocyte antigens as the recipient.

T cells may be obtained from a cell donor by routine methods including, for example, isolation from blood fractions, particularly the peripheral blood monocyte cell component, or from bone marrow samples.

Once T cells are obtained from the cell donor, a T cell which recognizes at least one epitope of a mucosally restricted antigen may be identified and isolated from the sample using standard techniques. The protein that comprises at least one epitope of a mucosally restricted antigen may be adhered to a solid support and contacted with the sample. T cells that remain on the surface after washing are then further tested to identify T cells that which recognize at least one epitope of a mucosally restricted antigen. Affinity isolation methods such as columns, labeled protein that binds to the cells, cell sorter technology may also be variously employed. T cells that recognize at least one epitope of a mucosally restricted antigen may also be identified by their reactivity in the presence of a protein with at least one epitope of a mucosally restricted antigen.

Once a T cell is identified as a T cell that recognizes at least one epitope of a mucosally restricted antigen, it may be clonally expanded using tissue culture techniques with conditions that promote and maintain cell growth and division to produce an exponential number of identical cells. The expanded population of T cells may be collected for administration to a patient.

In some embodiments, the cell donor is vaccinated prior to removal of a sample comprising T cells in order to induce an immune response against at least one epitope of a mucosally restricted antigen including a T cell immune response. In some embodiments, the cell donor is vaccinated prior to removal of a sample comprising T cells in order to induce an immune response against at least one epitope of a mucosally restricted antigen including a T cell immune response as part of a treatment which includes identifying and isolating a T cell that recognizes at least one epitope of a mucosally restricted antigen, culturing the cell to expand the number of such cells, and administering a plurality of such cells to a recipient who may be the same individual as the vaccinated cell donor (autographic procedure) or a different individual from the vaccinated cell donor (allographic procedure).

Examples of vaccines and vaccination methods that used may be used to induce T cells that comprise a T cell receptor which recognizes at least one epitope of a mucosally restricted antigen include those disclosed herein and those disclosed in the patents and published patent applications that have been incorporated by reference herein.

### T cells transformed with anti-CMA T cell receptors

A plurality of T cells which recognize at least one epitope of a mucosally restricted antigen may be obtained by isolating a T cell from a cell donor, transforming it with a nucleic acid molecule that encodes a T cell receptor which recognizes at least one epitope of a mucosally restricted antigen and, culturing the transformed cell to exponentially expand the number of transformed T cells to produce a plurality of such cells.

The cell donor may be the individual to whom the expanded population of cells will be administered, i.e. an autologous cell donor. Alternatively, the T cell may be obtained from a cell donor that is a different individual from the individual to whom the T cells will be administered, i.e. an allogenic T cell. If an allogenic T cell is used, it is preferred that the cell donor be type matched, that is identified as expressing the same or nearly the same set fo leukocyte antigens as the recipient.

T cells may be obtained from a cell donor by routine methods including, for example, isolation from blood fractions, particularly the peripheral blood monocyte cell component, or from bone marrow samples.

Once T cells are obtained from the cell donor, one or more T cells may be transformed with a nucleic acid that encodes a T cell receptor that recognizes at least one epitope of a mucosally restricted antigen.

The nucleic acid molecule that encodes the T cell receptor that recognizes at least one epitope of a mucosally restricted antigen may be obtained by isolating a T cell that recognizes at least one epitope of a mucosally restricted antigen from a "TCR gene donor" who has T cells that express a T cell receptor that recognizes at least one epitope of a mucosally restricted antigen. Such TCR gene donors may have T cells that recognize at least one epitope of a mucosally restricted antigen due to an immune response that arises from exposure to an immunogen other than by vaccination or, such TCR gene donors may be identified as those who have received a vaccine which induces production of T cells that recognize at least one epitope of a mucosally restricted antigen, i.e. a vaccinated TCR gene donor The vaccinated TCR gene donor may have been previously vaccinated or may be administered a vaccine specifically as part of an effort to generate such T cells that recognize at least one epitope of a mucosally restricted antigen for use in a method that comprises transforming T cells with a nucleic acid molecule that encodes a T cell receptor that recognizes at least one epitope of a mucosally restricted antigen, expanding the cell number, and administering the expanded population of transformed T cells to an individual.

The TCR gene donor may be the individual who will be the recipient of the transformed T cells or a different individual from the individual who will be the recipient of the transformed T cells. The TCR gene donor may be same individual as the cell donor or the TCR gene donor may be a different individual than the cell donor. In some embodiments, the cell donor is the recipient of the transformed T cells and the TCR gene donor is a different individual. In some embodiments, the cell donor is the same individual as the TCR gene donor and is a different individual from the recipient of the transformed T cells. In some embodiments, the cell donor is the same individual as the TCR gene donor and the same individual as the recipient of the transformed T cells.

Examples of vaccines that used may be used to induce T cells that comprise a T cell receptor which recognizes at least one epitope of a mucosally restricted antigen include those disclosed herein and those disclosed in the patents and published patent applications that have been incorporated by reference herein.

The nucleic acid molecule that encodes the T cell receptor that recognizes at least one epitope of a mucosally restricted antigen, i.e. the TCR coding sequence, may be a DNA or RNA molecule. The nucleic acid molecule may be operably linked to the regulatory elements necessary for expression of the TCR coding sequence in a donor T cell. In some embodiments, the nucleic acid molecule that comprises a TCR coding sequence is a plasmid DNA molecule. In some embodiments, the nucleic acid molecule that comprises a TCR coding sequence is a plasmid DNA molecule that is an expression vector wherein the TCR coding sequence is operably linked to the regulatory elements in the plasmid that are necessary for expression of the TCR coding sequence in a donor T cell. In some embodiments, a nucleic acid molecule that comprises a TCR coding sequence may be incorporated into viral particle which is used to infect a donor T cell. Packaging technology for preparing such particles is known. The TCR coding sequence incorporated into the particle may be operable linked to regulatory elements in the plasmid that are necessary for expression of the TCR coding sequence in a donor T cell. In some embodiments, the nucleic acid molecule that comprises a TCR coding sequence is incorporated into a viral genome. In some embodiments, the viral genome is incorporated into viral particle which is used to infect a donor T cell. Viral vectors for delivering nucleic acid molecules to cells are well known and include, for example, viral vectors based upon vaccine virus, adenovirus, adeno associated virus, pox virus as well as various retroviruses. The TCR coding sequence incorporated into the viral genome may be operable linked to regulatory elements in the plasmid that are necessary for expression of the coding sequence in a donor T cell.

Upon expression of the nucleic acid in the transformed T cells, the transformed cells may be tested to identify a T cell that recognizes at least one epitope of a mucosally restricted antigen. Such transformed T cells may be identified and isolated from the sample using standard techniques. The protein that comprises at least one epitope of a mucosally restricted antigen may be adhered to a solid support and contacted with the sample. T cells that remain on the surface after washing are then further tested to identify T cells that which recognize at least one epitope of a mucosally restricted antigen. Affinity isolation methods such as columns, labeled protein that binds to the cells, cell sorter technology may also be variously employed. T cells that recognize at least one epitope of a mucosally restricted antigen may also be identified by their reactivity in the presence of a protein with at least one epitope of a mucosally restricted antigen.

Once a T cell is identified as a T cell that recognizes at least one epitope of a mucosally restricted antigen, it may be clonally expanded using tissue culture techniques with conditions that promote and maintain cell growth and division to produce an exponential number of identical cells. The expanded population of T cells may be collected for administration to a patient.

### T cells transformed with anti-CMA membrane bound fusion proteins

A plurality of T cells which recognize at least one epitope of a mucosally restricted antigen may be obtained by isolating a T cell from a cell donor, transforming it with a nucleic acid molecule that encodes a CMA-binding membrane-bound fusion protein which includes a functional binding fragment of an antibody that binds to at least one epitope of a mucosally restricted antigen and a portion that renders the protein, when expressed in a cell such as a T cell, a membrane bound protein and, culturing the transformed cell to exponentially expand the number of transformed T cells to produce a plurality of such cells.

The cell donor may be the individual to whom the expanded population of cells will be administered, i.e. an autologous cell donor. Alternatively, the T cell may be obtained from a cell donor that is a different individual from the individual to whom the T cells will be administered, i.e. an allogenic T cell. If an allogenic T cell is used, it is preferred that the cell donor be type matched, that is identified as expressing the same or nearly the same set of leukocyte antigens as the recipient.

T cells may be obtained from a cell donor by routine methods including, for example, isolation from blood fractions, particularly the peripheral blood monocyte cell component, or from bone marrow samples.

Once T cells are obtained from the cell donor, one or more T cells may be transformed with a nucleic acid that encodes a CMA-binding membrane-bound fusion protein which includes a functional binding fragment of an antibody that binds to at least one epitope of a mucosally restricted antigen and a portion that renders the protein, when expressed in a cell such as a T cell, a membrane bound protein.

The nucleic acid molecule that encodes the CMA-binding membrane-bound fusion protein may be obtained by isolating a B cell that produces antibodies that recognize at least one epitope of a mucosally restricted antigen from an "antibody gene donor" who has such B cells that produce antibodies that recognizes at least one epitope of a mucosally restricted antigen. Such antibody gene donors may have B cells that produce antibodies that recognize at least one epitope of a mucosally restricted antigen due to an immune response that arises from exposure to an immunogen other than by vaccination or, such antibody gene donors may be identified as those who have received a vaccine which induces production of B cells that produce antibodies that recognize at least one epitope of a mucosally restricted antigen, i.e. a vaccinated antibody genetic donor The vaccinated antibody genetic donor may have been previously vaccinated or may be administered a vaccine specifically as part of an effort to generate such B cells that produce antibodies that recognize at least one epitope of a mucosally restricted antigen for use in a method that comprises transforming T cells with a nucleic acid molecule that encodes a CMA-binding membrane-bound fusion protein, expanding the cell number, and administering the expanded population of transformed T cells to an individual.

The antibody gene donor may be the individual who will be the recipient of the transformed T cells or a different individual from the individual who will be the recipient of the transformed T cells. The antibody gene donor may be same individual as the cell donor or the antibody gene donor may be a different individual than the cell donor. In some embodiments, the cell donor is the recipient of the transformed T cells and the antibody gene donor is a different individual. In some embodiments, the cell donor is the same individual as the antibody gene donor and is a different individual from the recipient of the transformed T cells. In some embodiments, the cell donor is the same individual as the antibody gene donor and the same individual as the recipient of the transformed T cells.

Examples of vaccines that used may be used to induce B cells that produce antibodies which recognize at least one epitope of a mucosally restricted antigen include those disclosed herein and those disclosed in the patents and published patent applications that have been incorporated by reference herein.

The nucleic acid molecule which encodes the CMA-binding membrane bound fusion protein comprises a coding sequence that encodes functional binding fragment of an antibody that recognizes at least one epitope of a mucosally restricted antigen linked to a protein sequence that provides for the expressed protein to be a membrane bound protein. The coding sequences are linked so that they encode a single product that is expressed.

The coding sequence that encodes a functional binding fragment of an antibody that recognizes at least one epitope of a mucosally restricted antigen may be isolated from a B cell from an antibody gene donor. Such a B cell may be obtained and the genetic information isolated. In some embodiments, the B cells are used to generate hybrid cells which express the antibody and therefore carry the antibody coding sequence. The antibody coding sequence may be determined, cloned and used to make the CMA-binding membrane-bound fusion protein. A functional binding fragment of an antibody that recognizes at least one epitope of a mucosally restricted antigen may include some or all of the antibody protein which when expressed in the transformed T cells retains its binding activity for at least one epitope of a mucosally restricted antigen.

The coding sequences for a protein sequence that provides for the expressed protein to be a membrane bound protein may be derived from membrane bound cellular proteins and include the transmembrane domain and, optionally at least a portion of the cytoplasmic domain, and/or a portion of the extracellular domain, and a signal sequence to translocate the expressed protein to the cell membrane.

The nucleic acid molecule that encodes the CMA-binding membrane-bound fusion protein that recognizes at least one epitope of a mucosally restricted antigen, i.e. the CMA-binding membrane-bound coding sequence, may be a DNA or RNA molecule. The nucleic acid molecule may be operably linked to the regulatory elements necessary for expression of the coding sequence in a donor T cell. In some embodiments, the nucleic acid molecule that comprises a CMA-binding membrane-bound coding sequence is a plasmid DNA molecule. In some embodiments, the nucleic acid molecule that comprises a CMA-binding membrane-bound coding sequence is a plasmid DNA molecule that is an expression vector wherein the coding sequence is operably linked to the regulatory elements in the plasmid that are necessary for expression of the CMA-binding membrane-bound coding sequence in a donor T cell. In some embodiments, a nucleic acid molecule that comprises a CMA-binding membrane-bound coding sequence may be incorporated into viral particle which is used to infect a donor T cell. Packaging technology for preparing such particles is known. The coding sequence incorporated into the particle may be operable linked to regulatory elements in the plasmid that are necessary for expression of the CMA-binding membrane-bound coding sequence in a donor T cell. In some embodiments, the nucleic acid molecule that comprises a CMA-binding membrane-bound coding sequence is incorporated into a viral genome. In some embodiments, the viral genome is incorporated into viral particle which is used to infect a donor T cell. Viral vectors for delivering nucleic acid molecules to cells are well known and include, for example, viral vectors based upon vaccine virus, adenovirus, adeno associated virus, pox virus as well as various retroviruses. The CMA-binding membrane-bound coding sequence incorporated into the viral genome may be operable linked to regulatory elements in the plasmid that are necessary for expression of the CMA-binding membrane-bound coding sequence in a donor T cell.

Upon expression of the nucleic acid in the transformed T cells, the transformed cells may be tested to identify a T cell that recognizes at least one epitope of a mucosally restricted antigen. Such transformed T cells may be identified and isolated from the sample using standard techniques. The protein that comprises at least one epitope of a mucosally restricted antigen may be adhered to a solid support and contacted with the sample. T cells that remain on the surface after washing are then further tested to identify T cells that which recognize at least one epitope of a mucosally restricted antigen. Affinity isolation methods such as columns, labeled protein that binds to the cells, cell sorter technology may also be variously employed. T cells that recognize at least one epitope of a mucosally restricted antigen may also be identified by their reactivity in the presence of a protein with at least one epitope of a mucosally restricted antigen.

Once a T cell is identified as a T cell that recognizes at least one epitope of a mucosally restricted antigen, it may be clonally expanded using tissue culture techniques with conditions that promote and maintain cell growth and division to produce an exponential number of identical cells. The expanded population of T cells may be collected for administration to a patient.

### Vaccines

Vaccines may be used to induce an immune response against one or more epitopes of a mucosally restricted antigen and produce TCR gene donors and/or donors of B cells useful to make CMA-binding membrane bound fusion proteins. A CD4+ helper epitope is provided to induce a broad based immune response. Examples of vaccines include, but are not limited to, the following vaccine technologies:
1) infectious vector mediated vaccines such as recombinant adenovirus, vaccinia, poxvirus, AAV, Salmonella, and BCG wherein the vector carries genetic information that encodes a chimeric protein that comprises at least an epitope of a mucosally restricted antigen, a CD4+ helper epitope, and optionally, a secretion signal, such that when the infectious vector is administered to an individual, the chimeric protein is expressed and a broad based immune response is induced that targets the mucosally restricted antigen;
2) DNA vaccines, i.e. vaccines in which DNA that encodes a chimeric protein that comprises at least an epitope of a mucosally restricted antigen, a CD4+ helper epitope, and optionally, a secretion signal, such that when the infectious vector is administered to an individual, the chimeric protein is expressed and a broad based immune response is induced that targets the mucosally restricted antigen;
3) killed or inactivated vaccines which a) comprise either killed cells or inactivated viral particles that display a chimeric protein that comprises at least an epitope of a mucosally restricted antigen and a CD4+ helper epitope, and b) when administered to an individual induces an immune response that targets the mucosally restricted antigen;
4) haptenized killed or inactivated vaccines which a) comprise either killed cells or inactivated viral particles that display a chimeric protein that comprises at least an epitope of a mucosally restricted antigen and a CD4+ helper epitope, b) are haptenized to be more immunogenic and c) when administered to an individual induces an immune response that targets the mucosally restricted antigen;
5) subunit vaccines which are vaccines that comprise a chimeric protein that comprises at least an epitope a mucosally restricted antigen and a CD4+ helper epitope; and
6) haptenized subunit vaccines which are vaccines that a) include a chimeric protein that comprises at least an epitope a mucosally restricted antigen and a CD4+ helper epitope and b) are haptenized to be more immunogenic.

### Mucosally restricted proteins

The mucosally restricted proteins are generally not expressed outside the mucosa. Accordingly, a systemic immune response targeting mucosally restricted proteins can be generated because the mucosally restricted proteins will be immunogenic with respect to at least some of the various components of the immune system when present outside the mucosa. That is, it will not be a self protein against which the immune system cannot elicit an immune response. Generally, mucosally restricted proteins are cellular proteins which are expressed in normal mucosa as well as cancer cells originating or otherwise derived from mucosal cells. Thus, the immune response against the mucosally restricted protein will recognize and attack cells outside the mucosa which express mucosally restricted protein such as metastatic cancer cells. Generally, the CD4+ immune response is either absent or significantly reduced when a mucosally restricted protein is introduced in tissue or body fluid outside of the mucosa.

Some examples of mucosally restricted proteins are cellular proteins include, but are not limited to, normally colorectal specific proteins such as guanylyl cyclase C, CDX-1, CDX-2, sucrase isomaltase, RELM beta (FIZZ2) (Holcomb IN, Kabakoff RC, Chan B, Baker TW, Gurney A, Henzel W, Nelson C, Lowman HB, Wright BD, Skelton NJ, Frantz GD, Tumas DB, Peale FV, Jr., Shelton DL, Hebert CC. FIZZ1, a novel cysteine-rich secreted protein associated with pulmonary inflammation, defines a new gene family. EMBO J 2000;19:4046-55.); Villin (also found in renal mucosa) (Wang Y, Srinivasan K, Siddiqui MR, George SP, Tomar A, Khurana S. A novel role for villin in intestinal epithelial cell survival and homeostasis. J Biol Chem 2008.), A33 (Johnstone CN, White SJ, Tebbutt NC, Clay FJ, Ernst M, Biggs WH, Viars CS, Czekay S, Arden KC, Heath JK. Analysis of the regulation of the A33 antigen gene reveals intestine-specific mechanisms of gene expression. J Biol Chem 2002;277:34531-9.), Lactase (lactase-phlorizin hydrolase) (Lee SY, Wang Z, Lin CK, Contag CH, Olds LC, Cooper AD, Sibley E. Regulation of intestine-specific spatiotemporal expression by the rat lactase promoter. J Biol Chem 2002; 277:13099-105.), H(+)/peptide cotransporter 1 (PEPT1, SLC15A1) (Daniel H. Molecular and integrative physiology of intestinal peptide transport. Annu Rev Physiol 2004;66:361-84; Terada T, Inui K. Peptide transporters: structure, function, regulation and application for drug delivery. Curr Drug Metab 2004;5:85-94; and Shimakura J, Terada T, Shimada Y, Katsura T, Inui K. The transcription factor Cdx2 regulates the intestine-specific expression of human peptide transporter 1 through functional interaction with Sp1. Biochem Pharmacol 2006;71:1581-8.); Intectin (Kitazawa H, Nishihara T, Nambu T, Nishizawa H, Iwaki M, Fukuhara A, Kitamura T, Matsuda M, Shimomura I. Intectin, a novel small intestine-specific glycosylphosphatidylinositol-anchored protein, accelerates apoptosis of intestinal epithelial cells. J Biol Chem 2004; 279:42867-74.); and Carbonic anhydrase (Drummond F, Sowden J, Morrison K, Edwards YH. The caudal-type homeobox protein Cdx-2 binds to the colon promoter of the carbonic anhydrase 1 gene. Eur J Biochem 1996; 236:670-81.)

Some examples of mucosally restricted proteins are cellular proteins include, but are not limited to, normally Breast-specific proteins such as Mammaglobin, (Watson MA, Fleming TP. Mammaglobin, a mammary-specific member of the uteroglobin gene family, is overexpressed in human breast cancer. Cancer Res 1996;56:860-5; Berger J, Mueller-Holzner E, Fiegl H, Marth C, Daxenbichler G. Evaluation of three mRNA markers for the detection of lymph node metastases. Anticancer Res 2006; 26:3855-60; Fleming TP, Watson MA. Mammaglobin, a breast-specific gene, and its utility as a marker for breast cancer. Ann N Y Acad Sci 2000;923:78-89.); B726P and small breast epithelial mucin (SBEM) (Miksicek RJ, Myal Y, Watson PH, Walker C, Murphy LC, Leygue E. Identification of a novel breast- and salivary gland-specific, mucin-like gene strongly expressed in normal and tumor human mammary epithelium. Cancer Res 2002;62:2736-40.)

Some examples of mucosally restricted proteins are cellular proteins include, but are not limited to, normally lung specific proteins such as LUNX (Iwao K, Watanabe T, Fujiwara Y, Takami K, Kodama K, Higashiyama M, Yokouchi H, Ozaki K, Monden M, Tanigami A. Isolation of a novel human lung-specific gene, LUNX, a potential molecular marker for detection of micrometastasis in non-small-cell lung cancer. Int J Cancer 2001;91:433-7; and Cheng M, Chen Y, Yu X, Tian Z, Wei H. Diagnostic utility of LunX mRNA in peripheral blood and pleural flu id in patients with primary non-small cell lung cancer. BMC Cancer 2008;8:156.) and TSC403 (Ozaki K, Nagata M, Suzuki M, Fujiwara T, Ueda K, Miyoshi Y, Takahashi E, Nakamura Y. Isolation and characterization of a novel human lung-specific gene homologous to lysosomal membrane glycoproteins 1 and 2: significantly increased expression in cancers of various tissues. Cancer Res 1998; 58:3499-503.).

### CD4+ T helper epitopes

Among the CD4+ helper epitopes that may be useful in making vaccines are those that form complexes with MHC Class II HLA serotypes HLA-DP, HLA-DQ and HLA-DR. Generally, self molecules will not form complexes to MHC Class II HLA and then, a complex, bind to CD4+ T cell receptors. Thus, the CD4+ helper epitopes are generally derived from a different species, most commonly a pathogenic species. CD4+ helper epitopes which form complexes to several types of MHC Class II HLA and then, a complex, bind to CD4+ T cell receptors are referred to as universal CD4+ helper epitopes.

Within each serotype, there are several types of each serotype. The MHC class II molecules are heterodimeric complexes. HLA-DP includes an α-chain encoded by HLA-DPA1 locus (about 23 alleles) and a β-chain encoded by HLA-DPB1 locus (about 127 alleles). Thus, there are about 2552 combinations for HLA-DP. HLA-DQ includes an α-chain encoded by HLA-DQA1 locus (about 34 alleles) and a β-chain encoded by HLA-DQB1 locus (about 86 alleles). Thus, there are about 1708 combinations for HLA-DQ. HLA-DR includes an α-chain encoded by HLA-DRA locus (about 3 alleles) and four (4) β-chains (for which any one person may be 3 possible per person), encoded by HLA-DRB1 (about 577 alleles), DRB3, DRB4, DRB5 loci (about 72 alleles). Thus, there are about 1398 combinations for HLA-DR. There are about 16 common types of HLA-DR (DR1-DR16).

Individuals may express some of the types but not others. Typically, individuals have multiple HLA types and the combination expressed by a particular individual, while perhaps not unique, defines a subset of the population as a whole. The identity of the types expressed by an individual may be routinely ascertained using well known and widely available technology. Thus, an individual may be "typed" to determine which types they express and are therefore involved in their immune responses.

A particular CD4+ helper epitope may be recognized by HLA Class II molecules that are present on one individual but not another. Accordingly, a product with an effective CD4+ helper epitope must be matched for the individual so that the product contains a CD4+ helper epitope recognized by an HLA type expressed on the individual's CD4+ T cells. Accordingly, an individual may be typed to determine MHC class II types present and then administered a vaccine that includes either multiple CD4+ helper epitopes including one or more of those that will be recognized by HLA type expressed by the individual or a vaccine that includes a CD4+ helper epitope that will be recognized by an HLA type expressed by the individual, i.e. that is matched to the individual.

Alternatively, a vaccine product may comprise a plurality of different chimeric proteins which collectively have CD4 epitopes which are recognized by all or many of the HLA types, thus increasing the probability that at least one will be effective in any given individual. Similarly, a vaccine product may contain a plurality of different chimeric genes encoding different chimeric proteins which collectively have CD4 epitopes which are recognized by all or many of the HLA types, thus increasing the probability that at least one will be effective in any given individual so that when administered to and expressed in an individual.

Thus, either the vaccine is matched for the individual or contains sufficient numbers of different CD4+ helper epitopes to assure recognition by an HLA type expressed a given individual's CD4+ T cells.

An alternative approach which allows for elimination of the need to match HLA types and the for elimination of the need to administer a plurality of possible matches provides a vaccine product that comprises a chimeric protein that includes a universal CD4+ helper epitope or a chimeric gene encoding a chimeric protein that includes a universal CD4+ helper epitope. A universal CD4+ helper epitope is a peptide sequence which is a match for and therefore recognized by multiple HLA types.

An example of a universal CD4+ helper epitope is a PADRE. The PADRE peptide forms complexes with at least 15 of the 16 most common types of HLA-DR. Since humans have at least one DR and PADRE binds to many of its types, PADRE has a high likelihood of being effective in most humans. In some embodiments, the CD4+ T cell epitopes are derived from the universal HLA-DR epitope PADRE (KXVAAWTLKA) (Alexander, J, delGuercio, MF, Maewal, A, Qiao L, Fikes J, Chestnut RW, Paulson J, Bundle DR, DeFrees S, and Sette A, Linear PADRE T Helper Epitope and Carbohydrate B Cell Epitope Conjugates Induce Specific High Titer IgG Antibody Responses, J. Immunol, 2000 Feb 1, 164(3):1625-33; Wei J, Gao W, Wu J, Meng K, Zhang J, Chen J, Miao Y. Dendritic Cells Expressing a Combined PADRE/MUC4-Derived Polyepitope DNA Vaccine Induce Multiple Cytotoxic T-Cell Responses. Cancer Biother Radiopharm 2008, 23:121-8; Bargieri DY, Rosa DS, Lasaro MA, Ferreira LC, Soares IS, Rodrigues MM. Adjuvant requirement for successful immunization with recombinant derivatives of Plasmodium vivax merozoite surface protein-1 delivered via the intranasal route. Mem Inst Oswaldo Cruz 2007, 102:313-7; Rosa DS, Iwai LK, Tzelepis F, Bargieri DY, Medeiros MA, Soares IS, Sidney J, Sette A, Kalil J, Mello LE, Cunha-Neto E, Rodrigues MM. Immunogenicity of a recombinant protein containing the Plasmodium vivax vaccine candidate MSP1(19) and two human CD4+ T-cell epitopes administered to non-human primates (Callithrix jacchus jacchus). Microbes Infect 2006, 8:2130-7; Zhang X, Issagholian A, Berg EA, Fishman JB, Nesburn AB, BenMohamed L. Th-cytotoxic T-lymphocyte chimeric epitopes extended by Nepsilon-palmitoyl lysines induce herpes simplex virus type 1-specific effector CD8+ Tc1 responses and protect against ocular infection. J Virol 2005; 79:15289-301 and Agadjanyan MG, Ghochikyan A, Petrushina I, Vasilevko V, Movsesyan N, Mkrtichyan M, Saing T, Cribbs DH. Prototype Alzheimer's disease vaccine using the immunodominant B cell epitope from beta-amyloid and promiscuous T cell epitope pan HLA DR-binding peptide. J Immunol 2005; 174:1580-6).

Universal CD4+ helper epitopes, such as PADRE and others are disclosed in U.S. Patent No. 5,736,142 issued April 7, 1998 to Sette, et al.; U.S. Patent No. 6,413,935 issued July 2, 2002 to Sette, et al.; and U.S. Patent No. 7,202,351 issued April 10, 2007 to Sette , et al. Other peptides reported to bind to several DR types include those described in Busch et al., Int. Immunol. 2, 443-451 (1990); Panina-Bordignon et al., Eur. J. Immunol. 19, 2237-2242 (1989); Sinigaglia et al., Nature 336, 778-780 (1988); O'Sullivan et al., J. Immunol. 147, 2663-2669 (1991) Roache et al., J. Immunol. 144, 1849-1856 (1991); and Hill et al., J. Immunol. 147, 189-197 (1991). Additionally, U.S. Patent No. 6,413,517 issued July 2, 2002 to Sette, et al. refers to the identification of broadly reactive DR restricted epitopes.

There are many known candidate proteins from which CD4+ T cell epitopes may be derived for use as a mucosally restricted antigen-fusion partner. Provided herein are examples of different proteins and different peptides which are examples of proteins which contain such CD4+ T cell epitopes. These proteins and peptides are intended to be non-limiting examples of CD4+ T cell epitopes.

In some embodiments, the CD4+ T cell epitope may be derived from tetanus toxin (Renard V, Sonderbye L, Ebbehoj K, Rasmussen PB, Gregorius K, Gottschalk T, Mouritsen S, Gautam A, Leach DR. HER-2 DNA and protein vaccines containing potent Th cell epitopes induce distinct protective and therapeutic antitumor responses in HER-2 transgenic mice. J Immunol 2003;171:1588-95; Moro M, Cecconi V, Martinoli C, Dallegno E, Giabbai B, Degano M, Glaichenhaus N, Protti MP, Dellabona P, Casorati G. Generation of functional HLA-DR*1101 tetramers receptive for loading with pathogen- or tumour-derived synthetic peptides. BMC Immunol 2005;6:24; BenMohamed L, Krishnan R, Longmate J, Auge C, Low L, Primus J, Diamond DJ. Induction of CTL response by a minimal epitope vaccine in HLA A*0201/DR1 transgenic mice: dependence on HLA class II restricted T(H) response. Hum Immunol 2000;61:764-79; and James EA, Bui J, Berger D, Huston L, Roti M, Kwok WW. Tetramer-guided epitope mapping reveals broad, individualized repertoires of tetanus toxin-specific CD4+ T cells and suggests HLA-based differences in epitope recognition. Int Immunol 2007;19:1291-301). In some embodiments, the CD4+ T cell epitope may be derived from Influenza hemagluttinin (Moro M, Cecconi V, Martinoli C, Dallegno E, Giabbai B, Degano M, Glaichenhaus N, Protti MP, Dellabona P, Casorati G. Generation of functional HLA-DR*1101 tetramers receptive for loading with pathogen- or tumour-derived synthetic peptides. BMC Immunol 2005;6:24).

In some embodiments, the CD4+ T cell epitope may be derived from Hepatitis B surface antigen (HBsAg) (Litjens NH, Huisman M, Baan CC, van Druningen CJ, Betjes MG. Hepatitis B vaccine-specific CD4(+) T cells can be detected and characterised at the single cell level: limited usefulness of dendritic cells as signal enhancers. J Immunol Methods 2008;330:1-11).

In some embodiments, the CD4+ T cell epitope may be derived from outer membrane proteins (OMPs) of bacterial pathogens (such as Anaplasma marginale) (Macmillan H, Norimine J, Brayton KA, Palmer GH, Brown WC. Physical linkage of naturally complexed bacterial outer membrane proteins enhances immunogenicity. Infect Immun 2008;76:1223-9). In some embodiments, the CD4+ T cell epitope may be derived from the VP1 capsid protein from enterovirus 71 (EV71) strain 41 (Wei Foo DG, Macary PA, Alonso S, Poh CL. Identification of Human CD4(+) T-Cell Epitopes on the VP1 Capsid Protein of Enterovirus 71. Viral Immunol 2008). In some embodiments, the CD4+ T cell epitope may be derived from EBV BMLF1 (Schlienger K, Craighead N, Lee KP, Levine BL, June CH. Efficient priming of protein antigen-specific human CD4(+) T cells by monocyte-derived dendritic cells. Blood 2000;96:3490-8; Neidhart J, Allen KO, Barlow DL, Carpenter M, Shaw DR, Triozzi PL, Conry RM. Immunization of colorectal cancer patients with recombinant baculovirus-derived KSA (Ep-CAM) formulated with monophosphoryl lipid A in liposomal emulsion, with and without granulocyte-macrophage colony-stimulating factor. Vaccine 2004;22:773-80; Piriou ER, van Dort K, Nanlohy NM, van Oers MH, Miedema F, van Baarle D. Novel method for detection of virus-specific CD4+ T cells indicates a decreased EBV-specific CD4+ T cell response in untreated HIV-infected subjects. Eur J Immunol 2005;35:796-805; Heller KN, Upshaw J, Seyoum B, Zebroski H, Munz C. Distinct memory CD4+ T-cell subsets mediate immune recognition of Epstein Barr virus nuclear antigen 1 in healthy virus carriers. Blood 2007;109:1138-46).

In some embodiments, the CD4+ T cell epitope may be derived from EBV LMP1 (Kobayashi H, Nagato T, Takahara M, Sato K, Kimura S, Aoki N, Azumi M, Tateno M, Harabuchi Y, Celis E. Induction of EBV-latent membrane protein 1-specific MHC class II-restricted T-cell responses against natural killer lymphoma cells. Cancer Res 2008;68:901-8).

In some embodiments, the CD4+ T cell epitope may be derived from HIV p2437, (Pajot A, Schnuriger A, Moris A, Rodallec A, Ojcius DM, Autran B, Lemonnier FA, Lone YC. The Th1 immune response against HIV-1 Gag p24-derived peptides in mice expressing HLA-A02.01 and HLA-DR1. Eur J Immunol 2007;37:2635-44).

In some embodiments, the CD4+ T cell epitope may be derived from Adenovirus hexon protein (Leen AM, Christin A, Khalil M, Weiss H, Gee AP, Brenner MK, Heslop HE, Rooney CM, Bollard CM. Identification of hexon-specific CD4 and CD8 T-cell epitopes for vaccine and immunotherapy. J Virol 2008;82:546-54). There are >30 identified CD4+ T cell epitopes for multiple MHC-II haplotypes, Vaccinia virus proteins (Calvo-Calle JM, Strug I, Nastke MD, Baker SP, Stern LJ. Human CD4+ T cell epitopes from vaccinia virus induced by vaccination or infection. PLoS Pathog 2007;3:1511-29) and >25 identified CD4+ T cell epitopes for multiple MHC-II haplotypes from 24 different vaccinia proteins.

In some embodiments, the CD4+ T cell epitopes are derived from heat shock protein (Liu DW, Tsao YP, Kung JT, Ding YA, Sytwu HK, Xiao X, Chen SL. Recombinant adeno-associated virus expressing human papillomavirus type 16 E7 peptide DNA fused with heat shock protein DNA as a potential vaccine for cervical cancer. J Virol 2000;74:2888-94.)

In some embodiments, the CD4+ T cell epitopes are derived from the Fc portion of IgG (You Z, Huang XF, Hester J, Rollins L, Rooney C, Chen SY. Induction of vigorous helper and cytotoxic T cell as well as B cell responses by dendritic cells expressing a modified antigen targeting receptor-mediated internalization pathway. J Immunol 2000;165:4581-91).

In some embodiments, the CD4+ T cell epitopes are derived from lysosome-associated membrane protein (Su Z, Vieweg J, Weizer AZ, Dahm P, Yancey D, Turaga V, Higgins J, Boczkowski D, Gilboa E, Dannull J. Enhanced induction of telomerase-specific CD4(+) T cells using dendritic cells transfected with RNA encoding a chimeric gene product. Cancer Res 2002;62:5041-8).

In some embodiments, the CD4+ T cell epitopes are derived from T helper epitope from tetanus toxin (Renard V, Sonderbye L, Ebbehoj K, Rasmussen PB, Gregorius K, Gottschalk T, Mouritsen S, Gautam A, Leach DR. HER-2 DNA and protein vaccines containing potent Th cell epitopes induce distinct protective and therapeutic antitumor responses in HER-2 transgenic mice. J Immunol 2003; 171:1588-95).

A sample of HLA haplotypes as well as representative CD4+ T cell epitopes for the indicated HLA molecule include, but are not limited to, the following:
HLA-DR*1101 - Tetanus Toxoid peptide residues 829-844, Hemagglutinin peptide residues 306-318 (Moro M, Cecconi V, Martinoli C, Dallegno E, Giabbai B, Degano M, Glaichenhaus N, Protti MP, Dellabona P, Casorati G. Generation of functional HLA-DR*1101 tetramers receptive for loading with pathogen- or tumour-derived synthetic peptides. BMC Immunol 2005;6:24.)
HLA-DRB1*0101 (DR1) - Tetanus Toxoid peptide residues 639-652, 830-843 or 947-967 and 14 other tetanus toxoid peptides (BenMohamed L, Krishnan R, Longmate J, Auge C, Low L, Primus J, Diamond DJ. Induction of CTL response by a minimal epitope vaccine in HLA A*0201/DR1 transgenic mice: dependence on HLA class II restricted T(H) response. Hum Immunol 2000;61:764-79; and James EA, Bui J, Berger D, Huston L, Roti M, Kwok WW. Tetramer-guided epitope mapping reveals broad, individualized repertoires of tetanus toxin-specific CD4+ T cells and suggests HLA-based differences in epitope recognition. Int Immunol 2007;19:1291-301).
HLA-DRB 1*0301 - EV71 VP1 residues 145-159 or 247-261 and 5 different tetanus toxoid peptides (Wei Foo DG, Macary PA, Alonso S, Poh CL. Identification of Human CD4(+) T-Cell Epitopes on the VP1 Capsid Protein of Enterovirus 71. Viral Immunol 2008; and James EA, Bui J, Berger D, Huston L, Roti M, Kwok WW. Tetramer-guided epitope mapping reveals broad, individualized repertoires of tetanus toxin-specific CD4+ T cells and suggests HLA-based differences in epitope recognition. Int Immunol 2007;19:1291-301).
HLA-DRB1*0405 - EV71 VP1 residues 145-159 or 247-261 (Wei Foo DG, Macary PA, Alonso S, Poh CL. Identification of Human CD4(+) T-Cell Epitopes on the VP1 Capsid Protein of Enterovirus 71. Viral Immunol 2008).
   HLA-DRB1*1301 - EV71 VP1 residues 145-159 or 247-261 (Wei Foo DG, Macary PA, Alonso S, Poh CL. Identification of Human CD4(+) T-Cell Epitopes on the VP1 Capsid Protein of Enterovirus 71. Viral Immunol 2008).
HLA-DR9 - Epstein Barr virus (EBV) latent membrane protein 1 (LMP1) residues 159-175 (Kobayashi H, Nagato T, Takahara M, Sato K, Kimura S, Aoki N, Azumi M, Tateno M, Harabuchi Y, Celis E. Induction of EBV-latent membrane protein 1-specific MHC class II-restricted T-cell responses against natural killer lymphoma cells. Cancer Res 2008;68:901-8).
HLA-DR53 - EBV LMP1 residues 159-175 (Kobayashi H, Nagato T, Takahara M, Sato K, Kimura S, Aoki N, Azumi M, Tateno M, Harabuchi Y, Celis E. Induction of EBV-latent membrane protein 1-specific MHC class II-restricted T-cell responses against natural killer lymphoma cells. Cancer Res 2008;68:901-8).
HLA-DR15 - EBV LMP1 residues 159-175 (Kobayashi H, Nagato T, Takahara M, Sato K, Kimura S, Aoki N, Azumi M, Tateno M, Harabuchi Y, Celis E. Induction of EBV-latent membrane protein 1-specific MHC class II-restricted T-cell responses against natural killer lymphoma cells. Cancer Res 2008;68:901-8).
HLA-DRB1*0401 - 15 different Tetanus Toxoid peptides (James EA, Bui J, Berger D, Huston L, Roti M, Kwok WW. Tetramer-guided epitope mapping reveals broad, individualized repertoires of tetanus toxin-specific CD4+ T cells and suggests HLA-based differences in epitope recognition. Int Immunol 2007;19:1291-301).
HLA-DRB1*0701 - 9 different Tetanus Toxoid peptides (James EA, Bui J, Berger D, Huston L, Roti M, Kwok WW. Tetramer-guided epitope mapping reveals broad, individualized repertoires of tetanus toxin-specific CD4+ T cells and suggests HLA-based differences in epitope recognition. Int Immunol 2007;19:1291-301).
HLA-DRB1*1501 - 7 different Tetanus Toxoid peptides (James EA, Bui J, Berger D, Huston L, Roti M, Kwok WW. Tetramer-guided epitope mapping reveals broad, individualized repertoires of tetanus toxin-specific CD4+ T cells and suggests HLA-based differences in epitope recognition. Int Immunol 2007;19:1291-301).
HLA-DRB5*0101 - 8 different Tetanus Toxoid peptides (James EA, Bui J, Berger D, Huston L, Roti M, Kwok WW. Tetramer-guided epitope mapping reveals broad, individualized repertoires of tetanus toxin-specific CD4+ T cells and suggests HLA-based differences in epitope recognition. Int Immunol 2007;19:1291-301).

### Secretion signals

Secreted antigens induce more potent CD4, CD8 and antibody responses following intramuscular immunization (Boyle JS, Koniaras C, Lew AM. Influence of cellular location of expressed antigen on the efficacy of DNA vaccination: cytotoxic T lymphocyte and antibody responses are suboptimal when antigen is cytoplasmic after intramuscular DNA immunization. Int Immunol 1997;9:1897-906; and Qiu JT, Liu B, Tian C, Pavlakis GN, Yu XF. Enhancement of primary and secondary cellular immune responses against human immunodeficiency virus type 1 gag by using DNA expression vectors that target Gag antigen to the secretory pathway. J Virol 2000;74:5997-6005.)

Generally, vaccines used to induce immune responses and produce T cells and B cells that recognize a mucosally restricted protein comprise secretion signals may be those involving nucleic acid based vaccines in which the coding sequence of the secretion signal is part of a chimeric gene that when expressed results in production of a fusion protein that includes a secretion signal. The presence of the secretion signal of such fusion proteins results in the transport and secretion of the expressed protein. In some embodiments, the secretion signals may be excised from the remainder of the fusion protein that comprises one or more mucosally restricted antigen epitopes and one or more CD4+ helper T epitopes upon secretion of the protein from the cell. In some embodiments, the fusion protein that comprises one or more mucosally restricted antigen epitopes and one or more CD4+ helper T epitopes is secreted from the cell with the secretion signal intact.

Secretion signals are well known and widely used in fusion and other recombinant proteins. One skilled in the art may readily select a known secretion signal which is functional in the species to which the vaccine is to be administered and design a chimeric gene that encodes a fusion protein that comprises a functional secretion signal, one or more mucosally restricted antigen epitopes and one or more CD4+ helper T epitopes.

Examples of secretion signals and their design are disclosed in vonHeijne G 1985 Signal sequences: the limits of variation J Mol Biol 184:99 and are general vonHeijne G 1990 Protein Targeting Signals Curr Opin Cell Biol 6:604. Further, Kuchler K and J Thorner 1992 Secretion of Peptides and Proteins Lacking Hydrophobic Signal Sequences: The Role of Adenosine Triphosphate-Driven Membrane Translocators Endocrine Reviews 13(3)499-514 discloses additional mechanisms by which proteins may be secreted.

In some embodiments, the mucosally restricted antigen is from a membrane bound cellular protein. Membrane bound cellular proteins often comprise an extracellular domain, a transmembrane domain and a cytoplasmic domain. In vaccines comprising one or more epitopes of a mucosally restricted antigen linked to one or more CD4+ T helper epitopes, the epitopes of a mucosally restricted antigen include some or all of an extracellular domain and, generally less than a complete transmembrane domain and no cytoplasmic domain. Such a fusion protein is transported such that the extracellular domain is translocated though the membrane but the transmembrane domain, to the extent that it is present, is not fully functional such that the protein is released from the cell.

### Nucleic acid-based vaccines

Some vaccines useful to induce immune responses that include T cells and B cells that recognize at least one epitope of a mucosally restricted protein comprise nucleic acid molecules which are administered to an individual whereby the nucleic acid molecules are taken up by cells of the individual and expressed to produce proteins encoded by the nucleic acid molecules. By producing protein within the individual's own cell, the protein can be processed to engage the cellular arm of the immune system and produced a broad, more effective immune response against the target immunogen.

Infectious vector mediated vaccines and DNA vaccines are vaccines that comprise nucleic acid molecules which are administered to an individual. Infectious vector mediated vaccines and DNA vaccines comprise nucleic acid molecules which include a chimeric gene that encodes a chimeric protein. The chimeric gene is operably linked to regulatory elements that are functional in the cell so that the chimeric protein is produced in at least some cells that take up the nucleic acid molecules of the vaccines.

The chimeric protein comprises: 1) at least one epitope of a mucosally restricted antigen, 2) a CD4+ helper epitope, and optionally, 3) a secretion signal. In such embodiments, the nucleic acid molecules are introduced into cells in the individual to whom the vaccine is administered where they are expressed to produce the chimeric protein in the cell. The intracellular production of the chimeric protein leads to a broad based immune response. In some embodiments, the chimeric additionally encodes secretion signal such that the chimeric protein includes a secretion signal. The chimeric protein that includes a secretion signal is processed by the cell for secretion. The secretion of chimeric protein sequences results in additional engagement of immune system processes and a broader based immune response.

Infection vectors generally refer to recombinant infectious vectors. Viral vectors and other vectors which infect cells and produce proteins within the cells are particularly effective since protein production within the cell is useful to engage intracellular processes involved in aspects of broad-based immune responses. Likewise, DNA vaccines are designed so that the DNA molecules, usually plasmids, are taken up by cells in the vaccinated individual. Protein sequences produced intracellularly may be used as targets in generating cellular immune responses such as through display of epitopes by MHC molecules to T cell receptors.

Examples of recombinant infectious vectors and technology includes, infectious vector mediated vaccines such as recombinant adenovirus, AAV vaccinia, Salmonella, and BCG. In each case, the vector carries a chimeric gene that encodes a chimeric protein.

As noted above, an advantage of a nucleic acid based vaccine is the intracellular production of the protein which comprises one or more epitope of a mucosally restricted antigen. The protein may be processed within the cell and presented in a manner to engage the cellular arm of immune system, resulting in a cellular immune response including cytotoxic T cells directed toward cells which display the one or more epitopes of a mucosally restricted antigen.

The presence of the CD4+ helper epitope provides for engagement of CD4+ immune cells in the immune response directed toward the one or more epitopes of a mucosally restricted antigen present on the chimeric protein. Without the CD4+ helper epitope the immune response against the one or more epitopes of a mucosally restricted antigen may restricted due to a lack of CD4+ immune cells specific for the one or more epitopes of a mucosally restricted antigen. By provided a CD4+ helper epitope together with the one or more epitopes of a mucosally restricted antigen, the immune response against the one or more epitopes of a mucosally restricted antigen may be broader and more complete by the simultaneous engagement of the CD4+ helper epitope that is recognized and capable of elicited a response by CD4+ immune cells of the individual. Thus a chimeric protein having a combination of one or more epitopes of a mucosally restricted antigen and a CD4+ helper epitope results in a much more effective immune response compared to that which would be elicited by the one or more epitopes of a mucosally restricted antigen without the CD4+ helper epitope.

The inclusion of the optional signal sequence may provide for further enhancement of the immune response directed at the one or more epitopes of a mucosally restricted antigen. The inclusion of the signal sequence in the chimeric protein will facilitate the export and secretion of the chimeric protein from the cell and into the extracellular milieu where the epitopes of chimeric protein can engage immune cells capable of recognizing them. This engagement may lead to a broader, more effective immune response and is significantly facilitated by the presence of the coding sequences on the chimeric gene for the signal sequence. Typically, the chimeric protein produced intracellularly from such a construct has the signal sequence which is removed as part of the secretion process, thus secreting a mature form of the chimeric protein which no longer includes the signal sequence.

The chimeric protein, which comprises at least an epitope of a mucosally restricted antigen, a CD4+ helper epitope and, optionally, a secretion signal is produced in the cell infected by the infectious vector. The mucosally restricted antigen epitopes present serve as targets for an immune response. The CD4+ helper epitope results in the engagement of CD4+ cell mediated immune responses. The secretion signal facilitates the secretion of the protein from the cell providing its presence extracellularly where it can serve as a target for various processes associated with different aspects of immune responses.

The one or more mucosally restricted antigen epitopes may be part of a full - length or truncated form of a mucosally restricted antigen. Some mucosally restricted antigens include signal sequences. Thus, the one or more mucosally restricted antigen epitopes may be part of a full-length or truncated form of a mucosally restricted antigen that includes the signal sequence of mucosally restricted antigen. The coding sequence of the CD4+ helper epitope would be linked to the coding sequence of the one or more mucosally restricted antigen epitopes such as a full-length or truncated form of a mucosally restricted antigen with the signal sequence such that expression of the chimeric protein results in the secretion of the mature chimeric protein which comprises the CD4+ helper epitope and one or more mucosally restricted antigen epitopes, such as a full-length or truncated form of a mucosally restricted antigen.

DNA vaccines are described in U.S. Patent No. 5,580,859, 5,589,466, 5,593,972, 5,693,622, and PCT/US90/01515, which are incorporated herein by reference. Others teach the use of liposome mediated DNA transfer, DNA delivery using microprojectiles (U.S. Pat. No. 4,945,050 issued Jul. 31, 1990 to Sanford et al., which is incorporated herein by reference). In each case, the DNA may be plasmid DNA that is produced in bacteria, isolated and administered to the animal to be treated. The plasmid DNA molecules are taken up by the cells of the animal where the sequences that encode the protein of interest are expressed. The protein thus produced provides a therapeutic or prophylactic effect on the animal.

The use of vectors including viral vectors and other means of delivering nucleic acid molecules to cells of an individual in order to produce a therapeutic and/or prophylactic immunological effect on the individual are similarly well known. Recombinant vaccines that employ vaccinia vectors are, for example, disclosed in U.S. Pat. No. 5,017,487 issued May 21, 1991 to Stunnenberg et al. which is incorporated herein by reference. Recombinant vaccines that employ poxvirus are, for example, disclosed in U.S. Pat. Nos. 5,744,141, 5,744,140, 5,514,375, 5,494,807, 5,364,773 and 5,204,243, which are incorporated herein by reference. Recombinant vaccines that employ adenovirus associated virus are, for example, disclosed in U.S. Pat. Nos. 5,786,211, 5,780,447, 5,780,280, 5,658,785, 5,474,935, 5,354,678, and 4,797,368, which are incorporated herein by reference. Recombinant vaccines that employ adenovirus associated virus are, for example, disclosed in U.S. Pat. Nos. 5,585,362, 5,670,488, 5,707,618 and 5,824,544, which are incorporated herein by reference.

### Killed or inactivated vaccines

Other forms of vaccines include killed or inactivated vaccines which may or may not be haptenized. The killed or inactivated vaccines may comprise killed cells or inactivated viral particles that display a chimeric protein that comprises at least an epitope of a mucosally restricted antigen and a CD4+ helper epitope. When administered to an individual, the killed or inactivated vaccines induce an immune response that targets the mucosally restricted antigen. Some killed or inactivated vaccines are haptenized. That is, they include an additional component, a hapten, whose presence increases the immune response against the killed or inactivated vaccines including the immune response against the one or epitope of a mucosally restricted antigen. The haptenized killed or inactivated vaccines comprise killed or inactivated vaccines which comprise either killed cells or inactivated viral particles that display a chimeric protein that comprises and a CD4+ helper epitope, and are haptenized. When administered to an individual, the killed or inactivated vaccines, or the haptenized killed or inactivated vaccines, an immune response that targets the mucosally restricted antigen is induced.

In some embodiments, cells that comprise at least one epitope of a mucosally restricted antigen and a CD4+ helper epitope are provided. In some embodiments the cells are human cells. In some embodiments the cells are non-human cells. In some embodiments the cells are bacterial cells. In some embodiments the cells are human cancer cells. Cells may be killed.

### Protein-based vaccines

Other forms of vaccines include subunit vaccines, including haptenized subunit vaccine. A subunit vaccine generally refers to a single protein or protein complex that includes an immunogenic target against which an immune response is desired. In the subunit vaccines herein comprise a chimeric protein that comprises at least an epitope a mucosally restricted antigen and a CD4+ helper epitope. The subunit vaccine may be haptenized to render the protein more immunogenic; i.e. the haptenization results in an enhanced immune response directed against the one or more epitopes of the mucosally restricted antigen.

The manufacture and use of subunit vaccines are well known. One having ordinary skill in the art can isolate a nucleic acid molecule that encodes CD4+ helper epitope linked to a mucosally restricted antigen or a fragment thereof. Once isolated, the nucleic acid molecule can be inserted it into an expression vector using standard techniques and readily available starting materials. The protein that comprises a CD4+ helper epitope linked a mucosally restricted antigen or a fragment thereof can be isolated.

The recombinant expression vector may comprises a nucleotide sequence that encodes the nucleic acid molecule that encodes the CD4+ helper epitope linked to the mucosally restricted antigen or a fragment thereof f. As used herein, the term "recombinant expression vector" is meant to refer to a plasmid, phage, viral particle or other vector which, when introduced into an appropriate host, contains the necessary genetic elements to direct expression of the coding sequence that encodes the protein. The coding sequence is operably linked to the necessary regulatory sequences. Expression vectors are well known and readily available. Examples of expression vectors include plasmids, phages, viral vectors and other nucleic acid molecules or nucleic acid molecule containing vehicles useful to transform host cells and facilitate expression of coding sequences. The recombinant expression vectors of the invention are useful for transforming hosts to prepare recombinant expression systems for preparing the isolated proteins of the invention.

Some embodiments relate to a host cell that comprises the recombinant expression vector. Host cells for use in well known recombinant expression systems for production of proteins are well known and readily available. Examples of host cells include bacteria cells such as E. coli, yeast cells such as S. cerevisiae, insect cells such as S. frugiperda, non-human mammalian tissue culture cells Chinese hamster ovary (CHO) cells and human tissue culture cells such as HeLa cells. In some embodiments, for example, one having ordinary skill in the art can, using well known techniques, insert such DNA molecules into a commercially available expression vector for use in these or other well known expression systems.

Some embodiments relate to a transgenic non-human mammal that comprises the recombinant expression vector that comprises a nucleic acid sequence that encodes the proteins used in the vaccine compositions. Transgenic non-human mammals useful to produce recombinant proteins are well known as are the expression vectors necessary and the techniques for generating transgenic animals. Generally, the transgenic animal comprises a recombinant expression vector in which the nucleotide sequence that encodes the CD4+ helper epitope linked to the mucosally restricted antigen or a fragment thereof operably linked to a mammary cell specific promoter whereby the coding sequence is only expressed in mammary cells and the recombinant protein so expressed is recovered from the animal's milk. One having ordinary skill in the art using standard techniques, such as those taught in U.S. Pat. No. 4,873,191 issued Oct. 10, 1989 to Wagner and U.S. Pat. No. 4,736,866 issued Apr. 12, 1988 to Leder, both of which are incorporated herein by reference, can produce transgenic animals which produce proteins that may be useful as or for making vaccines. Examples of animals are goats and rodents, particularly rats and mice.

In addition to producing these proteins by recombinant techniques, automated peptide synthesizers may also be employed to produce a protein that comprises the CD4+ helper epitopes linked to mucosally restricted antigen or a fragment thereof.. Such techniques are well known to those having ordinary skill in the art and are useful if derivatives which have substitutions not provided for in DNA-encoded protein production.

### Haptenization

In some embodiments, the vaccine is a protein that makes up a subunit vaccine or the cells or particles of a killed or inactivated vaccine. In some embodiments, such protein that makes up a subunit vaccine or the cells or particles of a killed or inactivated vaccine may be haptenized to increase immunogenicity. In some cases, the haptenization is the conjugation of a larger molecular structure to the mucosally restricted antigen or a fragment thereof or a protein that comprises the mucosally restricted antigen or a fragment thereof. In some cases, tumor cells from the patient are killed and haptenized as a means to make an effective vaccine product. In cases in which other cells, such as bacteria or eukaryotic cells which are provided with the genetic information to make and display the mucosally restricted antigen or a fragment thereof or a protein that comprises the mucosally restricted antigen or a fragment thereof, are killed and used as the active vaccine component, such cells are haptenized to increase immunogenicity. Haptenization is well known and can be readily performed.

Methods of haptenizing cells generally and tumor cells in particular are described in Berd et al. May 1986 Cancer Research 46:2572-2577 and Berd et al. May 1991 Cancer Research 51:2731-2734, which are incorporated herein by reference. Additional haptenization protocols are disclosed in Miller et al. 1976 J. Immunol. 117(5:1): 1591-1526.

Haptenization compositions and methods which may be adapted to be used to prepare haptenized immunogens according to the present invention include those described in the following U.S. Patents which are each incorporated herein by reference: U.S. Pat. No. 5,037,645 issued Aug. 6, 1991 to Strahilevitz; U.S. Pat. No. 5,112,606 issued May 12, 1992 to Shiosaka et al.; U.S. Pat. No. 4,526716 issued Jul. 2, 1985 to Stevens; U.S. Pat. No. 4,329,281 issued May 11, 1982 to Christenson et al.; and U.S. Pat. No. 4,022,878 issued May 10, 1977 to Gross. Peptide vaccines and methods of enhancing immunogenicity of peptides which may be adapted to modify immunogens of the invention are also described in Francis et al. 1989 Methods of Enzymol. 178:659-676, which is incorporated herein by reference. Sad et al. 1992 Immunolology 76:599-603, which is incorporated herein by reference, teaches methods of making immunotherapeutic vaccines by conjugating gonadotropin releasing hormone to diphtheria toxoid. Immunogens may be similarly conjugated to produce an immunotherapeutic vaccine of the present invention. MacLean et al. 1993 Cancer Immunol. Immunother. 36:215-22.2, which is incorporated herein by reference, describes conjugation methodologies for producing immunotherapeutic vaccines which may be adaptable to produce an immunotherapeutic vaccine of the present invention. The hapten is keyhole limpet hemocyanin which may be conjugated to an immunogen.

Vaccines according to some embodiments comprise a pharmaceutically acceptable carrier in combination with the active agent which may be, a nucleic acid molecule, a vector comprising a nucleic acid molecule such as a virus, a protein or cells. Pharmaceutical formulations are well known and pharmaceutical compositions comprising such active agents may be routinely formulated by one having ordinary skill in the art. Suitable pharmaceutical carriers are described in Remington's Pharmaceutical Sciences, A. Osol, a standard reference text in this field, which is incorporated herein by reference. The present invention relates to an injectable pharmaceutical composition that comprises a pharmaceutically acceptable carrier and the active agent. The composition is preferably sterile and pyrogen free.

In some embodiments, for example, the active agent can be formulated as a solution, suspension, emulsion or lyophilized powder in association with a pharmaceutically acceptable vehicle. Examples of such vehicles are water, saline, Ringer's solution, dextrose solution, and 5% human serum albumin. Liposomes and nonaqueous vehicles such as fixed oils may also be used. The vehicle or lyophilized powder may contain additives that maintain isotonicity (e.g., sodium chloride, mannitol) and chemical stability (e.g., buffers and preservatives). The formulation is sterilized by commonly used techniques.

An injectable composition may comprise the immunogen in a diluting agent such as, for example, sterile water, electrolytes/dextrose, fatty oils of vegetable origin, fatty esters, or polyols, such as propylene glycol and polyethylene glycol. The injectable must be sterile and free of pyrogens.

The vaccines may be administered by any means that enables the immunogenic agent to be presented to the body's immune system for recognition and induction of an immunogenic response. Pharmaceutical compositions may be administered parenterally, i.e., intravenous, subcutaneous, intramuscular.

Dosage varies depending upon the nature of the active agent and known factors such as the pharmacodynamic characteristics of the particular agent, and its mode and route of administration; age, health, and weight of the recipient; nature and extent of symptoms, kind of concurrent treatment, frequency of treatment, and the effect desired. An amount of immunogen is delivered to induce a protective or therapeutically effective immune response. Those having ordinary skill in the art can readily determine the range and optimal dosage by routine methods.

### Treatment methods

Aspects of the invention include methods of treating individuals who have cancer of a mucosal tissue. The treatment is provided systemically. By treating such an individual with a plurality of T cells that recognize at least one epitope of a mucosally restricted antigen as set forth herein, the T cells specifically targets the cancer cells expressing mucosal restricted antigens, particularly in the non-mucosal compartments of the individual's immune system. That is, the T cells will attack any cancer cells arising from mucosal tissue which are present outside the mucosa. The plurality of T cells that recognize at least one epitope of a mucosally restricted antigen are particularly useful to treat any metastatic disease including identified metastatic disease as well as any undetected metastasis, such as micrometastasis.

The plurality of T cells that recognize at least one epitope of a mucosally restricted antigen provide an adjuvant therapeutic treatment with the ordinary treatment provided upon diagnosis of cancer involving mucosal tissue and/or cancer vaccine treatment. One skilled in the art can diagnose cancer as cancer involving mucosal tissue. Detection of metastatic disease can be performed using routine methodologies although some minute level of cancer may be undetectable at the time of initial diagnosis of cancer. Typical modes of therapy include surgery, chemotherapy or radiation therapy, or various combinations. A plurality of T cells that recognize at least one epitope of a mucosally restricted antigen provide an additional weapon that selectively detects and eliminates cancer cells originating from the mucosal tissue but outside the mucosa due to metastasis.

Accordingly, in some embodiments, an individual is diagnosed as having cancer and the cancer is identified as originating from a type of mucosal tissue. Cancer of mucosal tissue may be diagnosed by those having ordinary skill in the art using art accepted clinical and laboratory pathology protocols. The identity of the specific type of mucosal tissue from which the cancer originated can be determined and a mucosally restricted antigen associated with such mucosal tissue type may be selected. A plurality of T cells that recognize at least one epitope of a mucosally restricted antigen is administered to the patient alone or as part of a treatment regimen which includes surgery, and/or radiation treatment and/or administration of other anti-cancer agents and/or administration of a cancer vaccine.

### Prophylactic methods

The plurality of T cells that recognize at least one epitope of a mucosally restricted antigen may also be used prophylactically in individuals who are at risk of developing as mucosal tissue cancer. There are several ways of indentifying individuals who are at elevated or particularly high risk relative to the population. Risk of some cancers can be predicted based upon family history and/or the presence of genetic markers. Certain behaviors or exposure to certain environmental factors may also place an individual into a high risk population. Previous diagnosis with primary disease which has been removed or in remission places the individual at higher risk. Those skilled in the art can assess the risk of an individual and determine whether or not they are at an elevated or high risk of mucosal tissue derived cancer.

Individuals who are at risk of developing as mucosal tissue cancer may be administered plurality of T cells that recognize at least one epitope of a mucosally restricted antigen prior to the individual having detectable disease.

### Compositions, Formulations, Doses and Regimens

A plurality of T cells that recognize at least an epitope of a mucosally restricted antigen according to some embodiments comprise a pharmaceutically acceptable carrier in combination with the cells. Pharmaceutical formulations comprising cells are well known and may be routinely formulated by one having ordinary skill in the art. Suitable pharmaceutical carriers are described in Remington's Pharmaceutical Sciences, A. Osol, a standard reference text in this field, which is incorporated herein by reference. The present invention relates to pharmaceutical composition for infusion.

In some embodiments, for example, the plurality of cells can be formulated as a suspension in association with a pharmaceutically acceptable vehicle. Examples of such vehicles are water, saline, Ringer's solution, dextrose solution, and 5% human serum albumin. The vehicle may contain additives that maintain isotonicity (e.g., sodium chloride, mannitol) and chemical stability (e.g., buffers and preservatives). The vehicle is sterilized prior to addition of cells by commonly used techniques.

The plurality of cells may be administered by any means that enables them to come into contact with cancer cells. Pharmaceutical compositions may be administered intravenously for example.

Dosage varies depending upon the nature of the plurality of cells, the age, health, and weight of the recipient; nature and extent of symptoms, kind of concurrent treatment, frequency of treatment, and the effect desired. Generally 1 x 10¹⁰ to 1 x 10¹² T cells are administered although more or fewer may also be administered, such as 1 x 10⁹ to 1 x 10¹³. Typically, 1 x 10¹¹ T cells are administered. The amount of cells delivered is the amount sufficient to induce a protective or therapeutically response. Those having ordinary skill in the art can readily determine the range and optimal dosage by routine methods.

The patents, published patent applications and references cited throughout this disclosure are hereby incorporated herein by reference.

The following example is provided as an exemplary embodiment only and is not intended to limit the scope of the invention.

### EXAMPLE

### Example 1

T cells may be harvested from PBMCs of colorectal cancer patients by leukapheresis or from tumor infiltratin lymphocytes (TILs) of colorectal cancer patients. TIL explants or PBMC-derived T cells will be cultured in complete medium (RPMI1640 based medium supplemented with 10% human serum) containing 6000 IU/ml of IL-2. The cultures may be maintained at cell concentrations between 5 x 10⁵ and 2 x 10⁶ cells per ml until several million TIL cells are available, usually 2-4 weeks. Multiple independent cultures may be screened by cytokine secretion assay for recognition of CMA epitopes. Two to six independent TIL cultures exhibiting the highest cytokine secretion may be further expanded in complete medium with 6000 IU per ml IL-2 until the cell number is over 5 x 10⁷ cells (this cell number is typically reached 3-6 weeks after tumor excision). TIL cultures that maintained CMA recognition will be expanded for treatment using one cycle of a rapid expansion protocol with irradiated allogeneic feeder cells, OKT3 (anti-CD3) antibody, and 6000 IU per ml IL-2. This rapid expansion protocol typically results in 1000-fold expansions of cells by the time of administration 14-15 days after initiation of the expansions. Patients may receive a bolus intravenous infusion of 1x10¹¹ cells over a 0.5 to 1 hour period.

### Example 2

T cells for engineering may be obtained from PBMCs following leukopherises by culturing cells at a concentration of 1 X 106/ml in T-cell culture medium AIM-V (Invitrogen Corp, Grand Isle, NY) with 300IU/ml IL-2, 100U/ml penicillin, 100µg/ml streptomycin, 1.25µg/ml amphotericin, 10µg/ml ciprofoloxicin, and 5% human AB serum supplemented with 50ng/ml OKT3. After 2 days of culture, cells will be collected, resuspended in fresh T cell culture medium without OKT3. A retroviral vector (such as pMSGV1) expressing either CMA-specific TCR α and β chains or a CMA-binding membrane bound fusion protein using a Murine Stem Cell Virus (MSCV) long terminal repeat (LTR) and a highly efficient internal ribosome entry site (IRES) derived from the human polio virus (for TCR only). A clinical grade retroviral vector supernatant will be commercially produced and used in a solid-phase transduction protocol that results in highly efficient gene transfer without the use of any selection method. The transduction of up to 5 X 10⁸ cells will be performed by overnight culture on Retronectin (CH-296, GMP grade Retronectin purchased from Takara Bio. Inc, Japan) coated, vector-preloaded six well tissue culture plates, using 6 ml vector and up to 5 X 10⁶ cells per well. Patients will receive a bolus intravenous infusion of 1x10¹⁰-10¹¹ cells over a 0.5 to 1 hour period.

### Example 3

CMA-reactive T cells will be expanded above from PBMCs or TILs. RNA isolated from a CMA-reactive T-cell clone will be subjected to RACE (rapid amplification of cDNA ends) polymerase chain reaction (PCR) and DNA sequence analysis in order to determine TCR α and β chain usage to design PCR primers for cloning of the individual chain full-length cDNAs. PolyA+RNA will be isolated from the T cells using the Poly (A) Pure mRNA purification kit (Ambion, Austin, TX). Reverse transcription-polymerase chain reaction (RT-PCR) was performed using the Titan One Tube RT-PCR kit (Roche, Indianapolis, IN) using pairs of oligonucleotide primers for the rearranged α and β TCR chains. The amplified products will be gel purified and cloned into the retroviral vector backbone. Cloned α and β segments will be confirmed by sequencing.

### Example 4

CMA-specific B cell hybridomas will be produced. Mice will be immunized with CMA to produce CMA-specific B cell (antibody) response. Spleens will be collected to harvest antibody producing B cells. These will be fused with the SP2/0-Agl4 myeloma cell line using a methylcellulose-based medium system, ClonaCell-HY Monoclonal Antibody Production Kit (StemCell Technologies, Inc.). Fused cells will be cloned by limiting-dilution and screened for CMA-specific antibody production to identity CMA-antibody producing hybridomas. These will be maintained as a permanent source of CMA-specific monoclonal antibody.

The heavy and light -chain antibody sequence will be cloned from the selected hybridoma to generate a scFV (single-chain Fv) antibody by PCR. cDNA will be produced from the hybridoma RNA using degenerate oligonucleotides (oligodT). The VL and VH (heavy and light -chain variable segments) will be amplified and assembled into the scFV using a three-step PCR approach with established oligonucleotides. The scFV produced from the CMA-specific hybridoma will be used to produce a chimeric antigen receptor (CMA-binding membrane-bound fusion protein or T-body).

The T-body genes will be of the tripartite configuration in which a CMA-specific scFv will be linked by PCR through the CD28 extracellular domain (from which the ligand-binding region was truncated) to the intracellular part of the FcRIy chain. The T-body cDNA construct will be cloned into the retroviral vector and used to transfect T cells to produce T-body-expressing T cells for therapy (above).

### Example 5

Transfer may be combined with various treatments including cytokine administration (primarily IL-2), CMA-directed vaccination and/or antibody therapy, chemotherapy, host preparative lymphodepletion with cyclophosphamide and fludarabine total-body irradiation (TBI), among other potential adjunct treatments.

### References

Dudley ME, Wunderlich JR, Robbins PF, Yang JC, Hwu P, Schwartzentruber DJ, Topalian SL, Sherry R, Restifo NP, Hubicki AM, Robinson MR, Raffeld M, Duray P, Seipp CA, Rogers-Freezer L, Morton KE, Mavroukakis SA, White DE, Rosenberg SA. Cancer regression and autoimmunity in patients after clonal repopulation with antitumor lymphocytes. Science 2002; 298:850-4.
Dudley ME, Yang JC, Sherry R, Hughes MS, Royal R, Kammula U, Robbins PF, Huang J, Citrin DE, Leitman SF, Wunderlich J, Restifo NP, Thomasian A, Downey SG, Smith FO, Klapper J, Morton K, Laurencot C, White DE, Rosenberg SA. Adoptive Cell Therapy for Patients With Metastatic Melanoma: Evaluation of Intensive Myeloablative Chemoradiation Preparative Regimens. J Clin Oncol 2008. 16.5449.
Morgan RA, Dudley ME, Wunderlich JR, Hughes MS, Yang JC, Sherry RM, Royal RE, Topalian SL, Kammula US, Restifo NP, Zheng Z, Nahvi A, de Vries CR, Rogers-Freezer LJ, Mavroukakis SA, Rosenberg SA. Cancer regression in patients after transfer of genetically engineered lymphocytes. Science 2006; 314:126-9.
Hughes MS, Yu YY, Dudley ME, Zheng Z, Robbins PF, Li Y, Wunderlich J, Hawley RG, Moayeri M, Rosenberg SA, Morgan RA. Transfer of a TCR gene derived from a patient with a marked antitumor response conveys highly active T-cell effector functions. Hum Gene Ther 2005; 16:457-72.
Pinthus JH, Waks T, Malina V, Kaufman-Francis K, Harmelin A, Aizenberg I, Kanety H, Ramon J, Eshhar Z. Adoptive immunotherapy of prostate cancer bone lesions using redirected effector lymphocytes. J Clin Invest 2004; 114:1774-81.
Yokoyama WM, Christensen M, Santos GD, Miller D. Production of monoclonal antibodies. Curr Protoc Immunol 2006; Chapter 2:Unit 2 5.
Snook AE, Stafford BJ, Li P, Tan G, Huang L, Birbe R, Schulz S, Schnell MJ, Thakur M, Rothstein JL, Eisenlohr LC, Waldman SA. Guanylyl Cyclase C-Induced Immunotherapeutic Responses Opposing Tumor Metastases Without Autoimmunity. J Natl Cancer Inst 2008; 100:950-961.
Irani Y, Tea M, Tilton RG, Coster DJ, Williams KA, Brereton HM. PCR amplification of the functional immunoglobulin heavy chain variable gene from a hybridoma in the presence of two aberrant transcripts. J Immunol Methods 2008; 336:246-50.
Laht S, Meerits K, Altroff H, Faust H, Tsaney R, Kogerman P, Jarvekulg L, Paalme V, Valkna A, Timmusk S. Generation and characterization of a single-chain Fv antibody against G, a hedgehog signaling pathway transcription factor. Hybridoma (Larchmt) 2008; 27:167-74.
Nam CH, Moutel S, Teillaud JL. Generation of murine scFv intrabodies from B-cell hybridomas. Methods Mol Biol 2002; 193:301-27.
Eshhar Z, Waks T, Bendavid A, Schindler DG. Functional expression of chimeric receptor genes in human T cells. Journal of Immunological Methods 2001; 248:67-76. Aspects and features of the present disclosure are set out in the following numbered clauses which contain the subject matter of the claims of the parent application as filed.

1. An isolated plurality of T cells which recognize at least one epitope of a mucosally restricted antigen.
2. The isolated plurality of T cells of clause 1 wherein said T cells are derived from clonal expansion of T cells isolated from an individual.
3. The isolated plurality of T cells of any of clauses 1 and 2 wherein said T cells are derived from clonal expansion of T cells which recognize at least one epitope of a mucosally restricted antigen that are isolated from an individual.
4. The isolated plurality of T cells of any of clauses 1 and 2 wherein said T cells are derived from clonal expansion of T cells that are isolated from an individual and transformed with a nucleic acid molecule which encodes a cancer mucosal antigen-binding membrane-bound fusion protein.
5. The isolated plurality of T cells of clause 4 wherein the nucleic acid molecule encodes a T cell receptor that isolated from a T cell which recognizes at least one epitope of a mucosally restricted antigen.
6. The isolated plurality of T cells of clause 4 wherein the cancer mucosal antigen-binding membrane-bound fusion protein encodes by the nucleic acid molecule comprises at least a functional antigen binding fragment of an antibody which recognizes at least one epitope of a mucosally restricted antigen.
7. The isolated plurality of T cells of any of clauses 1-6 that recognizes at least one epitope of a mucosally restricted antigen selected from the group consisting of guanylyl cyclase C, sucrase isomaltase, CDX1, CDX2, mammoglobin, and small breast epithelial mucin.
8. A pharmaceutical composition comprising an isolated plurality of T cells of any of clauses 1-7 and a pharmaceutically acceptable carrier or diluent.
9. A method of making a plurality of T cells that recognize at least one epitope of a mucosally restricted antigen comprising the steps of:
   isolating a sample from a cell donor that comprises at least one T cell that recognizes at least one epitope of a mucosally restricted antigen;
   identifying a T cell that recognize at least one epitope of a mucosally restricted antigen; and
   culturing said T cell under conditions to promote its replication for a period sufficient to produce a plurality of T cells that recognize a mucosally restricted antigen.
10. The method of clause 9 wherein the cell donor is administered a vaccine comprising a protein that comprises at least one epitope of a mucosally restricted antigen or a nucleic acid encoding a protein that comprises at least one epitope of a mucosally restricted antigen.
11. The method of clause 9 wherein the cell donor is administered a vaccine comprising a protein that comprises at least one epitope of a mucosally restricted antigen and a CD4 epitope or a nucleic acid encoding a protein that comprises at least one epitope of a mucosally restricted antigen and a CD4 epitope.
12. The method of any of clauses 9-11 wherein the T cell that recognize at least one epitope of a mucosally restricted antigen is a T cell that recognize at least one epitope of a mucosally restricted antigen selected from the group consisting of guanylyl cyclase C, sucrase isomaltase, CDX1, CDX2, mammoglobin, and small breast epithelial mucin.
13. The method of any of clauses 9-12 wherein the cell donor is a patient diagnosed with cancer of mucosal tissue or a patient identified as being at an elevated risk to develop cancer of mucosal tissue.
14. The method of clauses 13 wherein the cell donor is a patient identified as being at an elevated risk to develop cancer of mucosal tissue.
15. The method of clause 13 wherein the cell donor is a patient diagnosed with cancer of mucosal tissue.
16. The method of clause 13 wherein the cell donor is a patient diagnosed with metastatic cancer of mucosal tissue.
17. A method of making a plurality of T cells that recognize at least one epitope of a mucosally restricted antigen comprising the steps of:
   isolating a sample from a cell donor that comprises at least one T cell;
   transforming the T cell with a nucleic acid sequence that encodes either a T cell receptor that recognizes at least one epitope of a mucosally restricted antigen, or a cancer mucosal antigen-binding membrane-bound fusion protein that comprises at least a functional fragment of an antibody that binds to at least one epitope of a mucosally restricted antigen, wherein upon expression the fusion protein is a membrane bound protein; and
   culturing said transformed T cell under conditions to promote its replication for a period sufficient to produce a plurality of T cells that recognize a mucosally restricted antigen.
18. The method of clause 17 wherein the T cell is transformed with a nucleic acid sequence that encodes a T cell receptor that recognizes at least one epitope of a mucosally restricted antigen.
19. The method of clause 18 wherein the nucleic acid sequence that encodes a T cell receptor that recognizes at least one epitope of a mucosally restricted antigen is obtained by the steps of: administering a vaccine that comprises at least one epitope of a mucosally restricted antigen or a nucleic acid encoding a protein that comprises at least one epitope of a mucosally restricted antigen to a TCR gene donor, obtaining one or more T cells from the TCR gene donor, identifying a T cell comprises a T cell receptor that recognizes at least one epitope of a mucosally restricted antigen, isolating from the T cell a nucleic acid sequence that encodes T cell receptor that recognizes at least one epitope of a mucosally restricted antigen, and preparing an expression vector that comprises a nucleic acid sequence that encodes T cell receptor that recognizes at least one epitope of a mucosally restricted antigen.
20. The method of clause 19 wherein the TCR gene donor is administered a vaccine comprising a protein that comprises at least one epitope of a mucosally restricted antigen and a CD4 epitope or a nucleic acid encoding a protein that comprises at least one epitope of a mucosally restricted antigen and a CD4 epitope.
21. The method of clause 17 wherein the T cell is transformed with a nucleic acid sequence that encodes a cancer mucosal antigen-binding membrane-bound fusion protein that comprises at least a functional fragment of an antibody that binds to at least one epitope of a mucosally restricted antigen, wherein upon expression the fusion protein is a membrane bound protein.
22. The method of clause 21 wherein the nucleic acid sequence that encodes the cancer mucosal antigen-binding membrane-bound fusion protein is obtained by the steps of: administering a vaccine that comprises at least one epitope of a mucosally restricted antigen or a nucleic acid encoding a protein that comprises at least one epitope of a mucosally restricted antigen to a antibody gene donor, obtaining one or more B cells from the antibody gene donor, identifying either a B cell that produces an antibody which recognize at least one epitope of a mucosally restricted antigen or a hybrid cell derived from a B cell that produces antibodies which recognize at least one epitope of a mucosally restricted antigen, isolating from the B cell a nucleic acid sequence that encodes the antibody that recognizes at least one epitope of a mucosally restricted antigen, making a chimeric gene that encodes a functional fragment of the antibody that recognizes at least one epitope of a mucosally restricted antigen and a protein sequences that renders the fusion protein a membrane bound protein, and preparing an expression vector that comprises the chimeric gene.
23. The method of clause 22 wherein the vaccinated donor is administered a vaccine comprising a protein that comprises at least one epitope of a mucosally restricted antigen and a CD4 epitope or a nucleic acid encoding a protein that comprises at least one epitope of a mucosally restricted antigen and a CD4 epitope.
24. The method of any of clauses 17-23 wherein the T cell that recognize at least one epitope of a mucosally restricted antigen is a T cell that recognize at least one epitope of a mucosally restricted antigen selected from the group consisting of guanylyl cyclase C, sucrase isomaltase, CDX1, CDX2, mammoglobin, and small breast epithelial mucin.
25. The method of any of clauses 17-24 wherein the cell donor is a patient diagnosed with cancer of mucosal tissue or a patient identified as being at an elevated risk to develop cancer of mucosal tissue.
26. The method of clauses 25 wherein the cell donor is a patient identified as being at an elevated risk to develop cancer of mucosal tissue.
27. The method of clause 25 wherein the cell donor is a patient diagnosed with cancer of mucosal tissue.
28. The method of clause 25 wherein the cell donor is a patient diagnosed with metastatic cancer of mucosal tissue.
29. The method of any of clauses 17-28 wherein the TCR gene donor or antibody gene donor is a patient diagnosed with cancer of mucosal tissue or a patient identified as being at an elevated risk to develop cancer of mucosal tissue.
30. The method of clauses 29 wherein the cell donor is a patient identified as being at an elevated risk to develop cancer of mucosal tissue.
31. The method of clause 29 wherein the cell donor is a patient diagnosed with cancer of mucosal tissue.
32. The method of clause 29 wherein the cell donor is a patient diagnosed with metastatic cancer of mucosal tissue.
33. The method of any of clauses 17-23 wherein the cell donor is the TCR gene donor or antibody gene donor.
34. A method of treating an individual who has been diagnosed with cancer of a mucosal tissue comprising the step of administering to the individual an effective amount of a plurality of T cells which recognize at least one epitope of a mucosally restricted antigen produced by any of the methods of clause 13-20.
35. A method of preventing cancer of a mucosal tissue in an individual identified as being at an elevated risk of developing cancer of a mucosal tissue comprising the step of administering to the individual an effective amount of a plurality of T cells which recognize at least one epitope of a mucosally restricted antigen produced by any of the methods of clause 13-20.
36. A method of treating an individual who has been diagnosed with cancer of a mucosal tissue comprising the step of administering to the individual an effective amount of a plurality of T cells which recognize at least one epitope of a mucosally restricted antigen.
37. A method of preventing cancer of a mucosal tissue in an individual identified as being at an elevated risk of developing cancer of a mucosal tissue comprising the step of administering to the individual an effective amount of a plurality of T cells which recognize at least one epitope of a mucosally restricted antigen.
38. The method of any of clauses 36 and 37 wherein said T cells are derived from clonal expansion of T cells isolated from an individual.
39. The method of any of clauses 36-38 wherein said T cells are derived from clonal expansion of T cells which recognize at least one epitope of a mucosally restricted antigen that are isolated from an individual.
40. The method of any of clauses 36-38 wherein said T cells are derived from clonal expansion of T cells that are isolated from an individual and transformed with a nucleic acid molecule which encodes a protein that recognizes at least one epitope of a mucosally restricted antigen, wherein the protein is expressed as a membrane bound protein such that the T cells recognize at least one epitope of a mucosally restricted antigen.
41. The method of clause 40 wherein the nucleic acid molecule encodes a T cell receptor that isolated from a T cell which recognizes at least one epitope of a mucosally restricted antigen.
42. The method of clause 40 wherein the nucleic acid molecule encodes a cancer mucosal antigen-binding membrane-bound fusion protein which comprises at least a functional antigen binding fragments of an antibody which recognizes at least one epitope of a mucosally restricted antigen.
43. The method of any of clauses 36-42 that recognizes at least one epitope of a mucosally restricted antigen selected from the group consisting of guanylyl cyclase C, sucrase isomaltase, CDX1, CDX2, mammoglobin, and small breast epithelial mucin.
44. A nucleic acid molecule comprising a nucleotide sequence that encodes a protein which recognizes at least one epitope of a mucosally restricted antigen wherein the protein encodes a T cell receptor or a cancer mucosal antigen-binding membrane-bound fusion protein that comprises coding sequence for at least a functional fragment of an antibody which binds to at least one epitope of a mucosally restricted antigen and a portion which renders the fusion protein a membrane bound protein when the nucleotide sequence is expressed in a cell.
45. The nucleic acid molecule of clause 44 wherein the protein is a T cell receptor.
46. The nucleic acid molecule of clause 44 wherein the protein is a cancer mucosal antigen-binding membrane-bound fusion protein that comprises coding sequence for at least a functional fragment of an antibody which binds to at least one epitope of a mucosally restricted antigen and a portion which renders the fusion protein a membrane bound protein when the nucleotide sequence is expressed in a cell.
47. The nucleic acid molecule any of clauses 44-46 wherein the nucleotide sequence that encodes the cancer mucosal antigen-binding membrane-bound fusion protein is operatively linked to regulatory elements.
48. The nucleic acid molecule of any of clauses 44-46 wherein the nucleic acid molecule is DNA.
49. The nucleic acid molecule of clause 48 wherein the nucleic acid molecule is a plasmid.
50. The nucleic acid molecule of any of clauses 44-46 wherein the nucleic acid molecule is a viral genome.
51. The nucleic acid molecule of clause 50 wherein the nucleic acid molecule is a viral genome in a viral particle.
52. The nucleic acid molecule of clause 50 wherein the nucleic acid molecule is a viral genome in a viral particle selected from the group consisting of: adenovirus, AAV, poxvirus, SV40, and vaccinia.
53. The nucleic acid molecule of any of clauses 44-52 wherein the mucosally restricted antigen is selected from the group consisting of: guanylyl cyclase C, an immunogenically active fragment of guanylyl cyclase C, sucrase isomaltase, an immunogenically active fragment of sucrase isomaltase, CDX1, an immunogenically active fragment of CDX1, CDX2, an immunogenically active fragment of CDX2, mammoglobulin, an immunogenically active fragment of mammoglobin, small breast epithelial mucin, and an immunogenically active fragment of small breast epithelial mucin.
54. The nucleic acid molecule of any of clauses 44-52 wherein said chimeric protein comprises a mucosally restricted antigen selected from the group consisting of: guanylyl cyclase C, sucrase isomaltase, CDX1, CDX2, mammoglobin, and small breast epithelial mucin.
55. A T cell comprising a nucleic acid molecule of any of clauses 44-54.

## Claims

1. An isolated plurality of T cells which recognize at least one epitope of guanylyl cyclase C, wherein said T cells are derived from clonal expansion of T cells that are isolated from an individual and transformed with a nucleic acid molecule which encodes guanylyl cyclase C-binding membrane-bound fusion protein.

2. The isolated plurality of T cells of claim 1 wherein the guanylyl cyclase C - binding membrane-bound fusion protein encoded by the nucleic acid molecule comprises at least a functional antigen binding fragment of an antibody which recognizes at least one epitope of guanylyl cyclase C.

3. The isolated plurality of T cells of claim 1 or claim 2, wherein the T cells bind to and immunologically react with and against mucosal cancer cells that express the at least one epitope of guanylyl cyclase C.

4. The isolated plurality of T cells of any one of claims 1 to 3, wherein the nucleic acid molecule which encodes guanylyl cyclase C-binding membrane-bound fusion protein include the transmembrane domain and at least a portion of the cytoplasmic domain of a membrane bound cellular protein.

5. A composition comprising the plurality of T cells of any one of claims 1 to 4 for use in a method of treating an individual who has been diagnosed with cancer that expresses guanylyl cyclase C, the method comprising the step of administering to the individual an effective amount of said composition.

6. A composition comprising the plurality of T cells of any one of claims 1 to 4 for use in a method of preventing cancer that expresses guanylyl cyclase C in an individual identified as being at an elevated risk of developing cancer that expresses guanylyl cyclase C, the method comprising the step of administering to the individual an effective amount of said composition.

7. A method of making a plurality of T cells that recognize at least one epitope of guanylyl cyclase C, the method comprising the steps of:
a) transforming a T cell isolated from a cell donor with a nucleic acid sequence that encodes a guanylyl cyclase C-binding membrane-bound fusion protein, wherein upon expression the fusion protein is a membrane bound protein; and
b) culturing said transformed T cell under conditions to promote its replication for a period sufficient to produce a plurality of T cells that recognize guanylyl cyclase C.

8. The method of claim 7 wherein the guanylyl cyclase C -binding membrane-bound fusion protein encoded by the nucleic acid molecule comprises at least a functional antigen binding fragment of an antibody which recognizes at least one epitope of guanylyl cyclase C.

9. The method of claim 7 or claim 8, wherein the plurality of T cells that recognize guanylyl cyclase C bind to and immunologically react with and against mucosal cancer cells that express the at least one epitope of guanylyl cyclase C.

10. The method of any one of claims 7 to 9, wherein the nucleic acid molecule which encodes the guanylyl cyclase C-binding membrane-bound fusion protein includes the transmembrane domain and at least a portion of the cytoplasmic domain of a membrane bound cellular protein.

11. A nucleic acid molecule comprising a nucleotide sequence that encodes a guanylyl cyclase C-binding membrane-bound fusion protein that comprises a coding sequence for at least a functional fragment of an antibody which binds to at least one epitope of guanylyl cyclase C and a portion which renders the fusion protein a membrane bound fusion protein when the nucleotide sequence is expressed in a cell.

12. The nucleic acid of claim 11, wherein the guanylyl cyclase C-binding membrane-bound fusion protein binds to and immunologically reacts with and against mucosal cancer cells that express at least one epitope of guanylyl cyclase C when the nucleotide sequence is expressed in a cell.

13. The nucleic acid of claim 11 or claim 12, wherein the nucleic acid molecule encodes the transmembrane domain and at least a portion of the cytoplasmic domain of a membrane bound cellular protein.

14. The nucleic acid molecule of any one of claims 11 to 13 wherein the nucleotide sequence that encodes guanylyl cyclase C-binding membrane-bound fusion protein is operatively linked to regulatory elements and/or wherein the nucleic acid molecule is a plasmid or a viral genome.

15. A T cell comprising a nucleic acid molecule of any of claims 11 to 14.
